(19) Europäisches Patentamt
European Patent Office
Office européen des brevets

(11) **EP 3 577 491 B1**

(12) **EUROPEAN PATENT SPECIFICATION**

(45) Date of publication and mention
of the grant of the patent:
**04.10.2023 Bulletin 2023/40**

(21) Application number: **18709958.5**

(22) Date of filing: **26.02.2018**

(51) International Patent Classification (IPC):
*G01S 15/89* (2006.01)    *A61B 8/08* (2006.01)
*A61B 8/00* (2006.01)    *G01S 7/52* (2006.01)

(52) Cooperative Patent Classification (CPC):
**G01S 7/52049; A61B 8/0825; A61B 8/4472;**
**A61B 8/5253; G01S 7/52036; G01S 7/52071;**
**G01S 15/8915; G01S 15/8925; G01S 15/8979;**
**G01S 15/8993; G01S 15/8995**

(86) International application number:
**PCT/EP2018/054666**

(87) International publication number:
**WO 2018/154109 (30.08.2018 Gazette 2018/35)**

(54) **SYSTEM AND METHOD FOR SPEED AND ATTENUATION RECONSTRUCTION IN ULTRASOUND IMAGING**

SYSTEM UND VERFAHREN ZUR REKONSTRUKTION VON GESCHWINDIGKEIT UND DÄMPFUNG IN DER ULTRASCHALLBILDGEBUNG

SYSTÈME ET PROCÉDÉ DE RECONSTRUCTION DE VITESSE ET D'ATTÉNUATION D'IMAGERIE ULTRASONORE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **27.02.2017 EP 17158183**

(43) Date of publication of application:
**11.12.2019 Bulletin 2019/50**

(73) Proprietor: **ETH Zurich**
**8092 Zurich (CH)**

(72) Inventors:
• **GÖKSEL, Orcun**
**8044 Zürich (CH)**
• **SANABRIA, Sergio**
**8049 Zürich (CH)**

(74) Representative: **Toleti, Martin**
**E.Blum & Co. AG**
**Vorderberg 11**
**8044 Zürich (CH)**

(56) References cited:
**WO-A1-2015/091519**    **US-A1- 2002 099 290**
**US-A1- 2005 165 306**    **US-A1- 2013 041 260**

• **MICHAEL JAEGER ET AL: "Computed Ultrasound Tomography in Echo Mode for Imaging Speed of Sound Using Pulse-Echo Sonography: Proof of Principle", ULTRASOUND IN MEDICINE & BIOLOGY, vol. 41, no. 1, 1 January 2015 (2015-01-01), pages 235-250, XP055176008, ISSN: 0301-5629, DOI: 10.1016/j.ultrasmedbio.2014.05.019**

**Description**

Technical Field

The invention relates to a medical ultrasound system and to a related method.

Background of the Invention

Tumors and certain other anomalies in tissue are not always detectable in conventional B-mode ultrasound systems. However, these pathologies may present high contrast regarding other ultrasound characteristics, such as ultrasound propagation speed and attenuation. In order to obtain spatially-resolved images of these parameters, similarly to X-ray Computed Tomography (CT), ultrasound waves are transmitted through tissue and recorded at multiple angular directions, a family of methods known in the art as Ultrasound Computed Tomography (USCT). However, air (e.g., lungs) and bones behave as natural barriers to the propagation of ultrasound, limiting the applicability of ultrasound to soft tissue regions accessible from the skin (acoustic windows). Therefore, on the contrary of X-ray CT or Magnetic Resonance Imaging (MRI) full-body USCT is not feasible.

Medical applicability of USCT is currently limited mainly to the breast. This presently requires high-end application-specific Ultrasound Computed Tomography (USCT) equipment, based on a large number of stationary ultrasound sensors positioned around the breast, see "Breast density measurements with ultrasound tomography: A comparison with film and digital mammography", Duric et al., Med. Phys. 40 (1), 2013, or by mechanically rotating ultrasound sensors around the breast, see "Imaging of Sound Speed Using Reflection Ultrasound Tomography", Nebeker et al. Ultrasound Med 2012, 31, pp 1389-1404, both of which systems require the immersion of the breast in water and scanning it with a bulky custom-made ultrasound system. Though accurate results, such systems are burdensome in daily clinical use, they require additional space in the clinics and specialized personnel to perform, and they are typically costly and are constructed highly application-specific, in contrast to typical ultrasound systems that allow for wide-range of clinical uses.

In the method described in WO 2015/091519 A1 a Frequency Domain Reconstruction (here abbreviated as FDR) is proposed. Particularly, the Discrete Fourier Transform (DFT) $\Delta\tau(k_x, k_z, \varphi, \varphi_0)$ of the local echo phase shift $\Delta\tau(x, z, \varphi, \varphi_0)$ between two directions $\varphi$ and $\varphi_0$ over the image region $(x, z)$ is calculated for determining the local sound speed $c(x, z)$. Then, harmonic components $(k_x, k_z)$ of the slowness $\sigma(k_x, k_z)$ are determined in the frequency domain using a relation equivalent to:

$$\sigma(k_x, k_z) = T_{inv}\left(k_x', k_z', k_x, k_z, \varphi, \varphi_0\right)\Delta\tau(k_x, k_z, \varphi, \varphi_0)$$

wherein $\sigma(k_x, k_z)$ is the DFT of the slowness $\sigma(x, z)$, wherein the slowness $\sigma(x, z)$ is the reciprocal of the sound speed minus an average slowness, and $T_{inv}$ is a matrix. However, this method suffers from significant image reconstruction artefacts and a loss of resolution in the axial direction for moderate noise levels, which limit its applicability. As described in WO 2015/091519:

- The ill-posedness of the reconstruction results in low frequency artefacts that disturb the baseline of the bulk sound speed, as well as in high frequency artefacts that spread downwards through the image (page 22, Lines 14-17).
- With growing regularization of $T_{inv}$, artefacts due to the ill-posedness of the reconstruction are reduced, but "limited view artefacts", i.e. blurring of the upper and lower edges of the contrast regions and a drop of the base line adjacent to the left and right edges, become stronger, resulting in gradual loss of axial resolution, (page 22, Lines 17-25) .
- The local information can only be fully taken advantage of in the absence of noise when the regularization can be low. In practice, the local phase shift may be determined using echo phase tracking, which is prone to errors. With growing strength of regularization, the resulting image converges to an image with no axial resolution and with artifacts (page 23, Lines 12-13), (page 25, Lines 13-16).

- "Because of regularization, part of the spatial frequencies of the image are missing and the pixel gray level does not directly represent sound speed" (page 26, Lines 6-8).
- "The calculation of the calculation of the DFT requires that $\sigma(x, z)$ is defined over the full image region $(x, z)$. However, often in real tissue, strongly hypo-echoic regions such as large blood vessel lead to regions of missing data in the local echo phase shift maps, which, if not accommodated, lead to image reconstruction artifacts" (page 29 - Line 16-19) .

An in-depth analysis of this method associates the observed artefacts to an inappropriate regularization strategy, which limits the axial resolution, see "Computer ultrasound tomography in echo mode for imaging speed of sound using pulse-echo sonography: proof of principle", Jaeger et al. 2015, Ultrasound in Med. & Biol., Vol 41, No. 1, pages 235-250. The phase noise after sound-speed reconstruction results in a regular structure of high-spatial frequency streaks that run diagonally through the image, which is the main cause of loss of contrast in cases of low regularization. The regular structure of the image noise indicates that it is produced by an inappropriate regularization strategy.

In addition, document US 2002/0099290 A1 discloses A system and method for doing both transmission mode and reflection mode three-dimensional ultrasonic imagining. The multimode imaging capability may be used to provide enhanced detectability of cancer tumors within human breast, however, similar imaging systems are applicable to a number of other medical problems as well as a variety of non-medical problems in non-destructive evaluation (NDE).

Summary of the invention

The invention is defined by the independent claims 1 and 27.

The problem to be solved is therefore to enable a widespread use of ultrasound computed tomography (USCT). This problem is solved by an ultrasound transducer for emitting and receiving ultrasound, which ultrasound transducer is electrically connected to a processor. The processor is configured to determine an ultrasound based tomographic image subject to ultrasound waves received by the ultrasound transducer in response to ultrasound waves emitted by the ultrasound transducer and scattered and / or reflected by tissue to be investigated.

Hence, a single-sided inspection of an organ and/or tissue structure accessible is provided with conventional ultrasound. Single-sided means that the ultrasound transducer that preferably is a hand-held represents the sole ultrasound emitting and receiving entity, such that tissue to be investigated is approached by the ultrasound transducer from one side only. Preferably, the ultrasound transducer has a common housing for a set of emitter elements and a set of receiver elements for emitting and receiving ultrasound waves respectively. In particular, there is no man-made reflector involved in the measurements. Instead, the emitted ultrasound waves are scattered and / or reflected by tissue to be investigated, and in particular by structures in the tissue such as inclusions which represent one of spots or regions in the tissue with different ultrasound transmission characteristics than other regions in the tissue. Accordingly, it is preferred that the proposed system operates without any reflecting means other than the tissue itself.

Preferably, a novel image reconstruction method is applied, which can be built upon conventional hand-held ultrasound hardware. The minimum hardware required for implementing this method is the ultrasound transducer capable of emitting ultrasound waves and receiving and preferably recording ultrasound waves reflected and/or scattered at tissue structures, and the processor.

Preferably, the reconstruction method executed by the processor is based on misalignments in ultrasound parameters between images taken from different angles between the ultrasound transducer and the tissue to be investigated. Such misalignment, also referred to as mis-registration, in the ultrasound parameter, such as speed of sound, may be found in one or more or all areas of the investigated area of the tissue, each area referred to as cell, which cell is exposed to at least two ultrasound waves emitted from different angles. Preferably, in exposing the area of tissue to be investigated to ultrasound waves from different angles, paths of the ultrasound waves travel through at least partly different regions of the investigated area. Preferably, the regularization comprised in the reconstruction step is applied directly on the ultrasound parameters determined for each spatial region, i.e. cell, for the tissue, which spatial region preferably is a discrete area. Preferably, a spatial domain reconstruction is applied for image generation based on the mis-registrations detected and / or computed in the discretized problem space of the tissue.

For a single measurement from one angular direction with respect to the tissue, it is preferred to emit an ultrasound wave by one of the emitter elements, and receive and record the scattered and / or reflected ultrasound wave by all the receiver elements.

According to an aspect of the present invention, an ultrasound image reconstruction method is proposed, wherein a plurality of ultrasound signals is collected to allow access to set of cells in the imaged domain from paths passing through different domain regions, wherein the local mis-registrations between these plurality of ultrasound signals are determined, and wherein a spatial-domain reconstruction is employed based on computed mis-registrations in a discretized problem space.

Preferably, a system of equations is solved which relates to a discrete set of time measurements with speed of sound values in a discrete number of cells.

Preferably, the time measurements are expressed by a linear combination of the speed of sound values in the discrete number of cells.

Preferably, a system $\Delta t = L\sigma$ is solved wherein the weighted path lengths are assembled in a matrix $L$ of dimensions [MxC] and wherein the matrix $L$ represents geometric information that depends on the transmitter-receiver setup of the transducer and the size and shape of the cells, in particular their granularity/resolution. The speed of sound values per cell c are preferably assembled in the vector $\sigma$ of dimensions [Cx1], and the time measurements $\tau_m$ in the vector $\Delta t$ of dimensions [Mx1]. The overall number of time measurements M preferably is equal to or larger than the number of cells C for a determined linear system.

3

Preferably, prior physical information is incorporated, such as iteratively compensating wave refractions, wave path uncertainties, or others, preferably with a scheme in which the path measurements are iteratively actualized.

Preferably, prior information about lesion, aka tissue structures, is incorporated, such as location, size, delineation. For instance, a single speed of sound value is determined for a lesion region and a background region outside the lesion region each.

Preferably, acquisition of multi-static data is applied (every single element transmits sequentially, and all receiver signals are acquired), from which different path measurements can be generated (plane wave, synthetic aperture, focused...) .

Preferably, quantitative reconstruction is applied including procedures for estimated background speed of sound value and the quantitative speed of sound contrast in the inclusions. Preferably, observed timing differences with respect to an initial assumed speed of sound estimate may allow estimating these values.

A USCT method is proposed, which only requires single-sided access to the tissue to reconstruct ultrasound acoustic properties, and can preferably be implemented on a conventional medical ultrasound array, i.e. the ultrasound transducer, which can be hand-operated by a sonographer. Preferably, by combining different ultrasound transmitter excitations with image registration techniques, for instance speckle tracking, followed by a novel spatial domain reconstruction, local variations of speed of sound (SoS) in the tissue can be imaged.

The method is well suited to image organs in which only single-sided access is possible, such as liver, kidney, etc. However, it also significantly simplifies the clinical workflow for organs in which double-sided access is possible, in particular the breast, since no water bath, additional hardware elements are necessary to reconstruct USCT parameters.

The medical ultrasound system is used in a medical context: The medical ultrasound system may be used for one or more of medical screening, diagnosis, staging (e.g. of cancer), preoperative planning, intra-operative guidance, and post-operative follow-up.

Preferably, the ultrasound transducer is hand-held which is meant to be portable, or mobile. The ultrasound transducer can be held by a sonographer such as a doctor or a nurse during inspecting a patient.

The medical ultrasound system preferably is characterized by a Spatial Domain Reconstruction (SDR), for which time measurements are received and / or recorded by the ultrasound transducer for specific ultrasound wave propagation paths traversing a set of discretized tissue cells. Preferably, speed of sound variations in each of these cells are calculated from discretized relations between the speed of sound increments in individual tissue cells and the cumulative time measurements recorded by the transducer, given defined propagation paths. Thus the relations are defined and solved directly in an arbitrary spatial grid. With respect to previous art the following advantages are obtained:

- Spatial regularization can directly be applied in the spatial domain to solve the inverse problem, which allows for introducing available prior information about the reconstructed sound-speed distributions. For instance, it is possible to find the solution amongst the set of possible solutions that provides the best geometric delineation of inclusions (portions of the tissue that may be considered as tumorous), and the best accuracy for the sound-speed values in the inclusions. For instance, if the position and geometry of inclusions are a priori known (from instance from an ultrasound B-mode image), a reconstruction can be defined which determines the best sound-speed values in the inclusions.
- Quantitative speed-of-sound images, for example, are obtained in m/s, which provide a quantitative biomarker for tissue diagnosis and differentiation.
- The inverse problem can be solved, even if time measurements are only available for partial regions of the tissue domain and a harmonic decomposition of the image cannot be calculated. Similarly, the reliability of time measurements at specific tissue structures, for instance, expressed in terms of a correlation coefficient, can be directly incorporated into the regularization of the inverse problem.
- Arbitrarily complex path geometries can be easily incorporated with simple relations (scaling and additions), which allows defining arbitrary measurement configurations and correcting for physical effects such as wave refraction.

Other advantageous embodiments are listed in the dependent claims as well as in the description below.

## Brief Description of the Drawings

The embodiments defined above and further embodiments, features and advantages of the present invention can also be derived from the examples to be described hereinafter and are explained with reference to the annexed drawings, wherein:

FIG. 1 illustrates a measuring scenario including a medical ultrasound system according to an embodiment of the present invention;

FIG. 2 illustrates preferred strategies for the definition of a set of wave propagation paths $p$, from which time measurements $\tau_m$ can be extracted, according to embodiments of the present invention;

FIG. 3 illustrates different measurement approaches according to embodiments of the present invention;

FIG. 4 illustrates a medical ultrasound system according to an embodiment of the present invention;

FIGs. 5 to 7 shows measurement results of a medical ultrasound system according to an embodiment of the present invention;

FIG. 8 illustrates a method and a medical ultrasound system according to an embodiment of the present invention.

Detailed Description of the Drawings

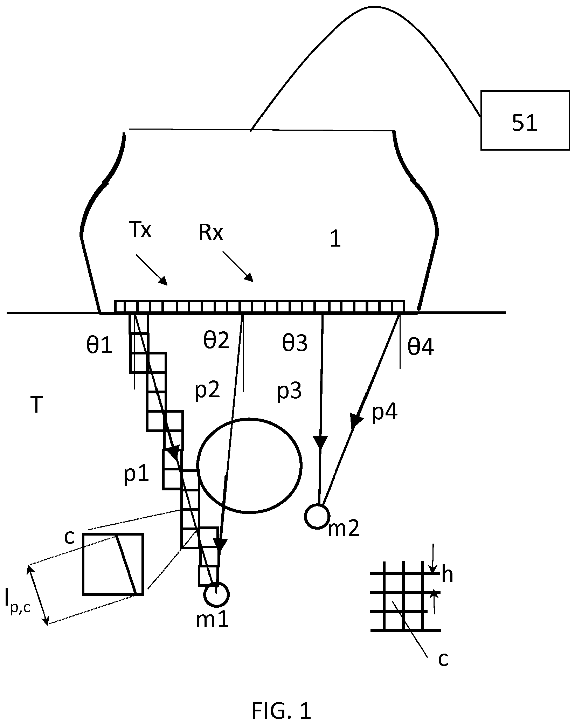

In a generic implementation of a medical ultrasound system illustrated in Fig.1, an ultrasound transducer 1 emits and receives ultrasound waves along a total of $P$ wave propagation paths $p$, corresponding to predefined trajectories, along which ultrasound waves propagate. Accordingly, the time delay $t_p$ is measured in form of a time difference between the time of emission of the ultrasound wave by one or more elements Tx of a set of emitter elements of the ultrasound transducer 1, and the time of receipt of the reflected ultrasound wave by one and preferably more and preferably all of elements Rx of a set of receiver elements, that may preferably be arranged in a row in the transducer 1. The transducer 1 accesses tissue T for investigation. This time delay $t_p$ is also referred to as time of flight. For tissue structures mx, either of natural or artificial nature, for which the position and/or geometry is accurately known, $t_p$ can be measured in absolute terms. In general, if the actual position of the tissue structures cannot be accurately determined, relative measurements $\tau_m$ can be obtained, which compare the time of flight along a predefined set of N paths $\{p_m\}=\{p_0, p_1 K\, p_N\}$, with $N \leq P$.

$$\tau_m = \sum_p w_{m,p} t_p \qquad\qquad \text{Eq. D.1}$$

The weights $w_{m,p}$ typically take the values +1 or -1 to express a relative measurement. Paths $p$ which are not part of the measurement m are zero weighted.

For instance, in the most simple version two distinct paths $j$ (test) and $i$ (reference), are compared for each measurement, in which case

$$\tau_m = t_j - t_i \qquad i,j \in \{p\}, \qquad i \neq j \qquad\qquad \text{Eq. D.2}$$

In this case, $w_{m,p} = 1$ for p = i, $w_{m,p} = -1$ for p = j, and $w_{m,p} = 0$ otherwise.

Preferably all paths converge to the same point or region in space.

Consequently, the same tissue position x,y preferably is accessed from two different wave propagation paths p1,p2 corresponding to two different angles θ1, θ2. The relative time measurements _m at coordinate x, y are preferably obtained from a timing shift observed in the images seen from these two angles, i.e. in the ultrasound waves received.

Preferably, a spatially-resolved speed-of-sound (SoS) image is determined based on multiple time measurements as expressed by Eq. D.1.

For this purpose, the tissue region insonified by the transducer is discretized into a finite number of cells c that reflect locations in the tissue. Without loss of generality, for a two-dimensional ultrasound system, for instance, it is preferred that a linear or a convex array of emitter and receiver elements is used so that the wave propagation is referred to a single plane defined by the radiation axis of the emitter and receiver elements, collectively referred to as transducer elements, and the tissue region is defined by one plane, in which the cells are located. The plane is then discretized into cells traversed by the paths $p$.

This cell structure supports the localization of portions / structures m of tissue that may be considered as tumorous, which portions are also referred to inclusions. The cell size is to be defined upfront and determines the resolution of the image. The process of determining the ultrasound parameter per cell based on the time of flight values is also referred to as reconstruction. Finally, a processor 51 of the medical ultrasound system is configured to convert the ultrasound parameter values as determined into an image that preferably is shown to medical personnel on a screen of the system. The conversion may include a coding of the ultrasound parameter values into colors, for example, or into grey scales.

With a path length $l_{p,c}$ [m] per ray path $p$ and *cell* c from the transmitter Tx to the reflecting structure m and back to the receiver Rx, the time of flight values $t_p$ are calculated in function of speed sound (SoS) increments $\sigma_c$ -also referred to as slowness increments, per cell $c$, i.e.:

$$t_p = \sum_{c=1}^{C} l_{p,c} \sigma_c \qquad \text{Eq. D.3}$$

This equation illustrates the time of flight values $t_p$ for a certain path $p$ as a sum of individual speed of sound values $\sigma_c$ per cell c, for the number of cells $C$ that are travelled along the subject $p$ path with the portion of the path length $l_{p,c}$ per individual cell $c$. Substituting Eq. D.3 in Eq. D.1 the relative time measurements $\tau_m$ are expressed in function of $\sigma_c$:

$$\tau_m = \sum_{\{pm\}} w_{m,p} \left( \sum_{c=1}^{C} l_{p,c} \sigma_c \right) = \sum_{c=1}^{C} \left( \sum_{p} w_{m,p} l_{p,c} \right) \sigma_c = \sum_{c=1}^{C} L_{m,c} \sigma_c \quad m = 1\text{K } M, M \geq C \qquad \text{Eq. D.4}$$

The last equality merges the path lengths $l_{p,c}$ and measurement weights $w_{m,p}$ in a single linear combination of weighted path lengths $L_{m,c}$, which is summarized with a matrix **L**.

The overall number of time measurement M preferably is equal to or larger than the number of cells C for a determined linear system. The system represented by equation D.4 can be expressed in matrix form for all measurements M and discretized cells C as $\Delta$**t** = **L**$\sigma$, wherein the weighted path lengths are assembled in a matrix **L** of dimensions [MxC] and represent geometric information that depends on the transmitter-receiver setup of the transducer and the size and shape of the cells, in particular their granularity/resolution. The speed of sound values $\sigma_c$ per cell c are assembled in the vector $\sigma$ of dimensions [Cx1], and the time measurements $\tau_m$ in the vector $\Delta$**t** of dimensions [Mx1].

In a first approximation, the path lengths $l_{p,c}$ for each ray path $p$ are calculated as the segment length traversing the cell $c$ (Fig. 1). For those skilled in the art, interpolation strategies are available, which can be utilized to improve the ray path representation on a discrete grid. These include nearest neighbor interpolation, linear interpolation and higher order interpolation kernels. By adding a certain kernel function or smoothness function to describe pressure distribution across the ray cross-section, the consistency and stability of the solvers is known to be enhanced. These kernel functions may be position dependent and obey both physical and computer graphics considerations.

## Path time measurement strategies

The inversion of Eq. D.4 requires the acquisition of relative time measurements $\tau_m$ which incorporate paths with respect to tissue structures, which can be identified in the ultrasound data. The recorded ultrasound signals can be described as a superposition of echoes at reflecting tissue structures *m1, m2, ...* In the most simple description, each echo is a delayed ultrasound wave. So if the echo corresponding to the same tissue structure can be identified for two different wave propagation paths, the relative time measurement $\tau_m$ can be obtained.

In a possible scenario, a series of echogenic features are identified in the recorded ultrasound signals. These can for example include natural geometric features (vessels, air/bone interfaces) or artificial features (microbubbles, catheters, biopsy needles, embedded reflectors). If the position and geometry of these features are well-defined, the problem reduces to a tracking problem, from which absolute time measurements can be obtained for each path. If the position and geometry of the reflecting features is not accurately known (for instance, due to the unknown speed-of-sound distribution), relative measurements that contain the same feature can still be obtained along different wave propagation paths, which is known in several contexts by "multipath" or "spatial diversity". Image registration procedures well known in the literature can then be applied to align the reflectivity patterns obtained from different path directions. The local mis-registration residuals provides relative time measurements for these paths.

In another possible scenario, which is preferred for soft tissue (for instance breast, liver, muscle, myocardium), the recorded ultrasound signals comprise of a superposition of a larger number of microscopic features (for instance, individual tissue cells), which converge into homogeneous textured patterns or speckle, typically showing Rayleigh distribution. Each tissue region is known to show a unique speckle tracking. Speckle tracking (ST) algorithms are well-known in the ultrasound elastography literature, which provide "best texture match" between two displaced / scaled / rotated versions of the same speckle patterns, allowing to determine displacements in the echo signals. Some well-known algorithms are Digital Image Correlation and Optic Flow. These algorithms allow the registration of speckle patterns obtained from different path directions.

In a preferred version, which is implemented for the illustrated examples, a correlation measurement is defined between two different speckle patterns:

$$c_{yx}[m,n] = \frac{r_{x-\bar{x},y-\bar{y}}[m;n]}{\sigma_x \sigma_y} = \frac{\sum_{k=-W/2}^{W/2}\left(y[m+n+k]-\bar{y}[m;n]\right)\left(x^*[m+k]-\bar{x}[m]\right)}{\sqrt{\sigma_y[m,n]\sigma_x[m]}}$$

$$\sigma_y[m,n] = \sum_{k=-W/2}^{W/2}\left|y[m+n+k]-\bar{y}[m;n]\right|^2$$

$$\sigma_x[m] = \sum_{k=-W/2}^{W/2}\left|x^*[m+k]-\bar{x}[n]\right|^2 \qquad \text{Eq. T.1}$$

$$\bar{x} = (W+1)^{-1}\sum_{k=-W/2}^{W/2}x^*[m+k] \qquad \bar{y} = (W+1)^{-1}\sum_{k=-W/2}^{W/2}y[m+n+k]$$

$$m = 0...M-1, \quad n = -S...S$$

in which two discretized one-dimensional ultrasound signals *x[m]* (reference) and *y[m]* (test) containing the same misaligned speckle patterns are compared. *x* and *y* are associated to two different path configurations containing echoes of common speckle patterns. The sample index *m* corresponds to discretized time samples of the signal recorded by the ultrasound receiver, or in general to any other time-distance scale. S is a defined search window in which the signal match is found. If available, prior information can be incorporated to the search window. *W* is the correlation block size, or number of samples uses for texture matching. For each sample *m* and possible displacement n, $c_{yx}[m;n]$ is the normalized correlation coefficient, which provides a measurement of linear relationship between the two signals, irrespective of echo energy / scale. The scale factors $\sigma_x$ and $\sigma_y$ correspond to the variances of the signals *x* and *y*. With Eq. T.1 the optimum displacement at each tissue position *n* is calculated as:

$$n_{opt}[m] = \arg\max_m c_{y,x}[m,n] \qquad \text{Eq. T.2}$$

The displacement measurements $n_{opt}$ obtained at different tissue positions *m,* scaled in time units, provide a set of time measurements $\tau_m$ which can be used as input data for equation Eq. D.4.

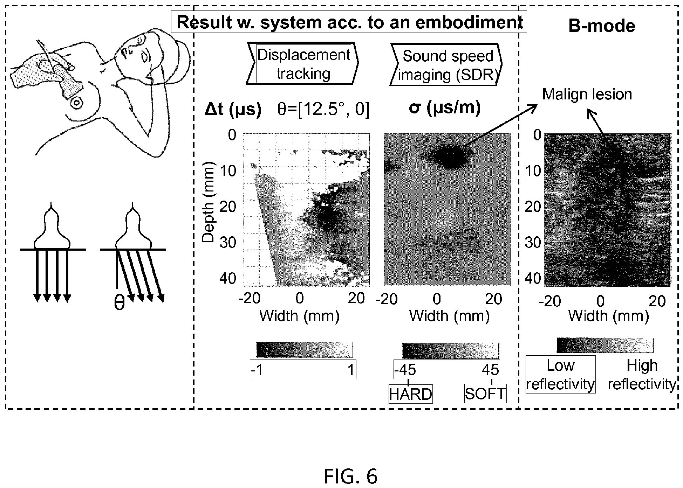

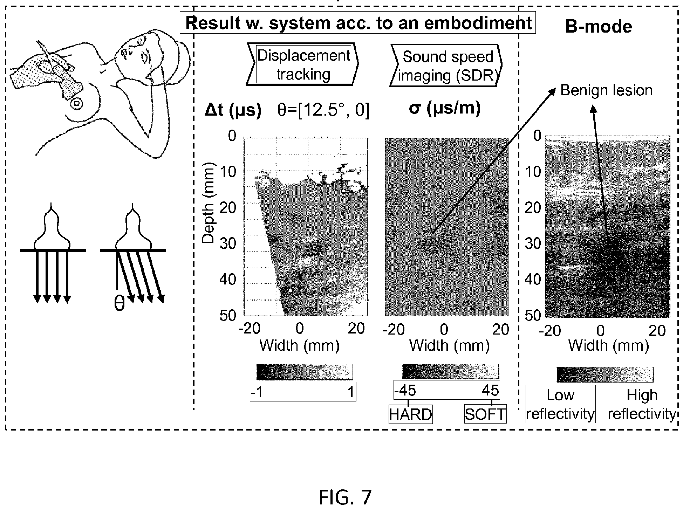

Eq. T.1 may be extended to signals of higher dimensional order $x[m_1, m_2...]$, $y[m_1, m_2...]$, for which higher order correlations are performed. For instance (as exemplified below in Figure 6 and Figure 7), two-dimensional correlations may be performed by simultaneously considering ultrasound signal traces corresponding to adjacent receiver elements or by considering adjacent beam formed lines.

A quality measurement $q_m$ for the registration, can be obtained at each *m* by calculating $q_m = c_{yx}[m, n_{opt}]$

, which provides the correlation between the two matched texture patterns. $c_{yx}[m, n_{opt}]$ ranges between -1...1, with the largest positive values providing the best correlation. Several physical mechanisms (for instance low tissue echogeneicity) can lead to loss of speckle coherence and poor registration quality. A threshold can be defined, below which displacement measurements are discarded in Eq. D.4.

Even if time measurements are not available in a wide range of tissue cell positions *m,* speed of sound values can still be reconstructed for these cells *c,* as long as sufficient measurements are available for a determined system $\Delta t = L\sigma$. This is illustrated with an example in FIG 1. Even if the measurements m1 and m2 are obtained outside the tumorous inclusion region, the compared paths {p1, p2} for m1 traverse the inclusion and allow differentiating it with respect to m2 {p3, p4}, where the inclusion is not insonified. This is an important advantage of the presented invention with respect to prior FDR art, in which time measurements m1, m2 are required at all calculated cell positions c1, c2 to be able to perform a harmonic decomposition of σ.

In a more general scenario the quality measurement (negative values are zeroed) or a transformation of it can be incorporated into the solution term Eq D.4 to regularize the solution term. For instance, if the optimum solution is expressed as a minimization process $\hat{\sigma} = \arg\min_\sigma\left\{\left\|\Delta t - L\sigma\right\|_2\right\}$, the quality measurements $q_m$ for each measurement m can be cast into a vector **q** of dimensions [M × 1] and the solution can be expressed as:

$$\hat{\boldsymbol{\sigma}} = \arg\min_{\sigma} \left\{ \left\| \mathbf{q}^T \mathbf{I}(\Delta\mathbf{t} - \mathbf{L}\boldsymbol{\sigma}) \right\|_2 \right\} \qquad \text{Eq T.3}$$

where $\mathbf{I}$ is the identity matrix of rank M. Potential for such an inclusion of observation quality into the image reconstruction is another advantage of the presented invention.

The simple texture matching described here can be generalized to incorporate regularization information, for instance, physical and geometric constraints given by the way the matched patterns are generated (see next section) or continuity constraints. The registration can then be defined as an optimization problem based on the minimization cost function, which simultaneously incorporates the information of all measurements $m$. Such cost can also be minimized using discrete and graph-based optimization techniques well-known to those skilled in the art, such as graph-cuts, Markov-random Fields, and Conditional Random Fields. Outlier detection algorithms, for instance Random sample consensus (RANSAC) can be used to filter out undesired structures from the time measurements. Texture matching redundancies between different path combinations may also be exploited to improve speckle tracking quality.

**Path definition strategies:**

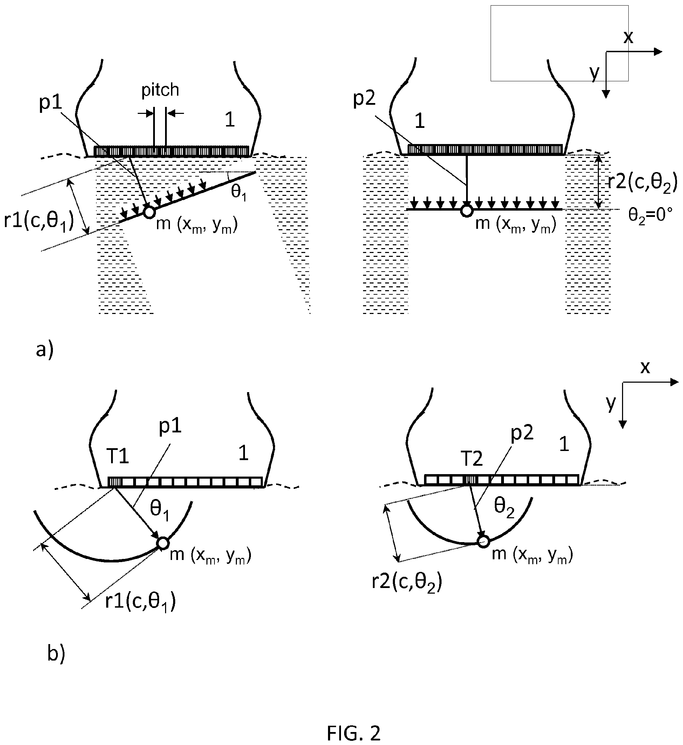

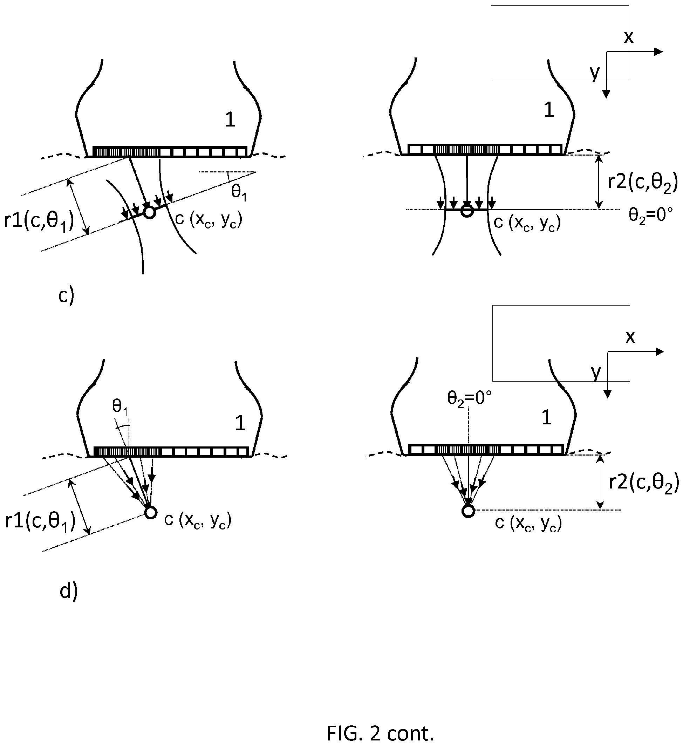

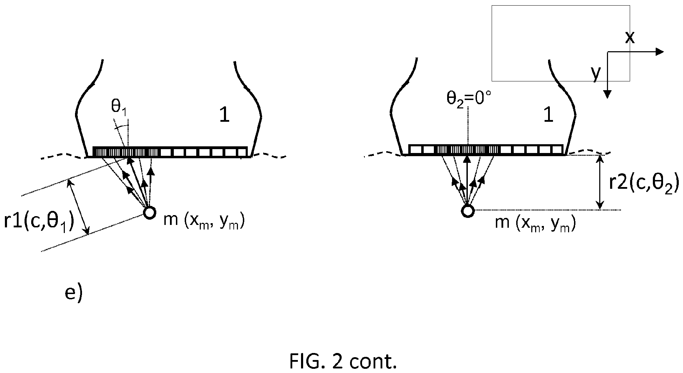

Figure 2 exemplifies preferred strategies for definition of a set of wave propagation paths $p$, from which time measurements $\tau_m$ can be extracted according to some of the methods outlined above. Even if the Spatial Domain Reconstruction (SDR) allows for arbitrary path comparisons, certain path properties are preferred. Sufficient diversity should be provided to solve the inverse problem Eq T.3. For instance, it is well-known from the theory of computed tomography reconstruction that the conditioning of the reconstruction improves if the reconstructed cells are traversed by a wide range of path directions. The defined paths preferably highlight the matched tissue features or patterns, so that accurate measurements $q_m$ can be obtained in the registration step. Incremental differences between paths are also more bound to provide a good matching quality than larger differences. An organized path definition in function of a few simple parameters $\theta_i$ - i.e. the angle of the path in the plane e.g. relative to a longitudinal axis of the ultrasound transducer, or relative to a horizontal axis of the ultrasound transduced , e.g. represented by a linear row of transducer elements - that can be adjusted in the ultrasound transducer is also desired to simplify the measurement process, as well as to optimize measurement speed.

In a preferred embodiment, a single transducer with N transducer elements is operated in "multi-static" or "full matrix" mode, with each element individually firing and the rest receiving. This concept is equivalent to ultrasound imaging with transmitter and receiver aperture of one element. This provides a total of $N \times N$ time traces for the transducer and provides the most generic representation of a linear tissue model. The multi-static matrix can be represented as $A(t, tx, rx)$, where $t$ is the time coordinate, and tx and $rx$ are respectively the transmit and receive indexes. The firing process can be optimized for real time operation, for instance, by simultaneously recording all receive channels $rx$ for each firing element $tx$. The signal to noise ratio can be also be optimized by coding the signals before transmission. The coding can be performed in either or both time and spatial domains, and allows to measure the multi-static matrix with significantly improved signal to noise ratio.

Figure 2 illustrates several examples of path definition strategies. Non-focused wavefronts can be generated to achieve a large tissue coverage in front of the ultrasound transducer 1. Non-focused wavefronts are plane wavefronts or full aperture wavefronts, which are shown in Fig. 2a as white areas embraced by a dashed area representing areas not exposed to the ultrasound wavefront, and synthetic aperture or single-element circular wavefronts are shown in Fig 2b by means of an arrow.

In the case of plane wavefronts (Fig. 2a), spatial diversity can be obtained by controlling the angle of incidence $\theta$ of the plane wavefront. Consequently, the same tissue position m can be accessed from two different wave propagation paths (p1, p2) corresponding to two different angles ($\theta 1$, $\theta 2$) and a time measurement $\tau_m$ can be obtained by subtracting the time difference between the two paths p1, p2. Plane wavefronts are synthesized by firing all elements of the ultrasound transducer (full aperture) with controlled transmit delays between transmit elements to achieve a coherent wavefront. For instance, for a linear array of transducer elements with element separation *pitch* as pictured in Fig. 2a and a plane wavefront of angle $\Theta 1$, the transmitter delays for element index tx are adjusted as:

$$\text{delay(tx)} = tx*pitch*\sin(\theta 1)/v_B,$$

with $v_B$ being the average speed of sound in the inspected tissue. Details of $v_B$ calculation are described below. $v_B$ can be estimated to an accuracy of 1% or better with known global image registration methods. This leads to a typical precision of 0.1° or better in the generated angle $\Theta 1$, which can be iteratively refined from the speed-of-sound reconstruction results. Plane wavefronts $B(t, \theta, rx)$ of arbitrary angle $\theta$ can be synthesized from a recorded multi-static matrix by time-shifting and scaling and summing the recorded tx traces for each rx element:

$$B(t, \theta, rx) = \sum_{tx} apodization(tx)A(t - delay(tx), tx, rx) \qquad \text{Eq. P.1}$$

It should be noted that, due to their angulation, plane wavefronts provide only limited coverage of the tissue domain. The dashed regions in Fig. 2 are "shadow regions", which are not accessible by the plane wavefront. If two such plane wavefronts $\Theta 1$ and $\Theta 2$ are compared to obtain a time measurement at point m, such point m preferably is within the coverage region of both plane wavefronts.

Circular wavefronts can be generated by firing one element at a time (Fig. 2b). In this case a full coverage of the tissue region in front of the transducer 1 is obtained while typically there is no shadow region in the tissue. In this case, the same transmit delay can be applied for all transmit elements. Spatial diversity is obtained by comparing two paths p1 or p2 of circular wavefronts generated by two different transducer elements T1 and T2 representing respective paths p1 and p2 with corresponding angles $\Theta 1$ and $\Theta 2$ with respect to the same tissue position m.

Clutter may complicate time measurements $\tau_m$ if the matched tissue structures are overlapped with echoes from other reflecting tissue structures, which coincide with the reflections of these structures. This problem can be addressed by collimating the transmitted ultrasound energy in a localized beam that propagates only through a defined region of the tissue. For the plane wavefronts described in Fig. 2a this can be achieved by limiting the aperture of the transmitted plane wavefront, as it is illustrated in Fig. 2c. A natural collimation of the plane wavefront is obtained at the near field distance $NF = Aperture^2/4/v_B*freq$, where the Aperture is expressed in distance units [m], and $freq$ is the frequency of the transmitted ultrasound pulses. Starting from the multistatic matrix A($t$, $tx$, $rx$) and applying Eq. P.1 for limited apertures, collimated wavefronts can be obtained at arbitrary tissue positions. Collimation as well reduces the extent of "shadow regions", especially in the region close to the transducer emitting surface (near field).

Another possibility to reduce clutter is to generate a focused ultrasound beam at defined measurement points $m$, so that tissue reflectivity is maximized in a small region around the desired measurement point $m$. In this case the time delays of the transmit elements are adjusted to achieve a constructive interference at the position m: $delay$(tx) = $-distance$(tx, m)/vB, where $distance$(tx, $m$) is the geometric distance between the transmit element Tx and the measurement point $m$. Spatial diversity can in this case be achieved by synthesized focused ultrasound beams with different steering angles $\Theta 1$ and $\Theta 2$, as illustrated in Fig 2d. Focused ultrasound beams at an arbitrary tissue position can be generated from the multistatic matrix A(t, tx, rx) by applying Eq. P.1. with the aforementioned $delay(tx)$. A full aperture of finite size can be used to generate focused ultrasound beams. Undesired physical effects, such as side-lobes or near-field interferences, can be minimized by ensuring a constant ratio #f = $depth/aperture$, or by scaling the signals of the transmit aperture with an apodization function $apodization(tx)$, for instance a Hanning, Hamming or Gaussian window. Apodization can also be applied to the collimated plane wavefronts described in Fig. 2c.

Circular wavefronts with increased signal to noise ratio may also be generating by firing multiple elements at a time, and combining their contributions with Eq. P.1. This concept is known to those skilled in the art as "virtual sources". The center of the circular source is in this case defined by focusing the ultrasound waves at a position m in front or behind the transducer radiation surface. The same mechanism may be used to achieve collimated sources in the transducer elevation plane. In general, several techniques are known for improving the signal to noise ratio by firing multiple elements with different transmit pulses, for instance, by using different delays, apodizations (including positive/negative/zero- sign), or different pulse functions.

So far, spatial diversity and clutter reduction have been described in terms of the ultrasound transmit process. However, the ultrasound receive process can also be controlled to provide additional spatial diversity and clutter reduction. This is illustrated in Fig. 2e. Similarly to Eq. P.1., by delaying and summing the ultrasound signals recorded by individual receiver elements, focusing and steering with respect to specific points $m$ are achieved. Eq P.1 is then generalized as Eq. P.2:

$$B(m, \theta_{tx}, \theta_{rx}) = \sum_{rx} \sum_{tx} apodization(tx, rx)A(t(m) - delay(tx) - delay(rx), tx, rx) \qquad \text{Eq. P.2}$$

Eq. P.2 computes tissue reflectivity patterns for a set of measurement points $m$ in function of diversity parameters defined in transmission $\theta_{tx}$ and reception $\theta_{rx}$. Each $B(m)$ can be understood as a beamformed ultrasound image. If an additional demodulation process is applied to $B(m)$, it results in a so-called conventional ultrasound B-mode image. Therefore, ultrasound B-mode images obtained in function of different diversity parameters $\theta$ can be registered to obtain relative time measurements $\tau_m$ and subsequently to reconstruct the speed of sound distribution with Eq. D.4. However, in general, beamformed ultrasound images (radio-frequency RF or demodulated IQ data) are preferred, since the phase (time) can be tracked more precisely from the carrier frequency.

If the speed of sound distribution is constant at all inspected tissue regions and the average speed of sound $v_B$ is accurately known, then all $B(m, \theta_{tx}, \theta_{rx})$ provide the same image $B(m, \theta_{tx}, \theta_{rx})=B(m)$. If this is not the case, small misregistrations are observed between the images $B(m, \theta_{tx}, \theta_{rx})$, which can be determined with the matching algorithms described in the previous section. Expressed in time units, the misregistrations (apparent motion / displacements) between two arbitrary images $B(m, \theta_1)$ and $B(m, \theta_2)$ provide relative time measurements $\tau_m$, which enable the reconstruction of the speed of sound perturbations $\sigma_c$ at each cell with respect to the assumed nominal values $v_B$.

Given a recorded multi-static matrix and sufficient computational resources, all techniques described above can be applied to the multi-static matrix $A(t, tx, rx)$ to obtain a large number of time measurements $\tau_m$, which can be then used to solve the unknown speed of sound distribution from Eq. D.4. Following the synthetic aperture concept illustrated in Fig. 2b, the theoretical maximum number of diversity paths for a given measurement point $m$, assuming that the point can be resolved, is given by the number of recorded transmit-receive combinations NxN. The maximum number of measurement points $m$ over a defined tissue region is given by a discretization of the tissue region by the best achievable spatial resolution with ultrasound waves, which is typically an order of magnitude larger than the wavelength $lambda = v_B/f$, with f being the dominating frequency of the ultrasound signals. This as well corresponds to the best spatial resolution of the cells c for the reconstructed speed-of-sound distribution $\sigma$. Therefore, a maximum of NxN measurements is theoretically available for each cell c, which provides a highly overdetermined system Eq. D.4, in which robust reconstructions can be achieved.

Three-dimensional reconstructions can be achieved for 2D-matrix array probes, other transducer constructs with more than one element placed or imaging in the out-of-plane direction. These allow for an extension of the spatial diversity strategies outlined in Figure 2. For instance, arbitrarily oriented plane wavefronts can be obtained (both in plane and out of plane inclination). Both spherical waves (single element fired) and cylindrical waves (a row or columns of elements simultaneously fired) can be used for spatial diversity. Similar considerations to those above regarding the maximum number of diversity paths apply.

In one example, relative time measurements m are performed at each cell c by comparing plane wavefronts at angles $\theta1=20°$ and $-20°$ with respect to $\theta2=0°$. Preferably, the $\tau_m$ values were generated using a ray tracing algorithm, so that no uncertainties due to speckle tracking or unaccounted wave propagation paths are present. Random noise was then added to the $m$ matrix, following a Gaussian distribution with zero mean and a standard deviation expressed as percentage with {1,2,5,10,20,50,56,63}% of the peak $\tau_m$ value for each example, which correspond to a signal-to-noise ratio of $\{\tau40,34,26,20,14,6,5,4\}$ dB.

**Incorporation of physical wave propagation into reconstruction**

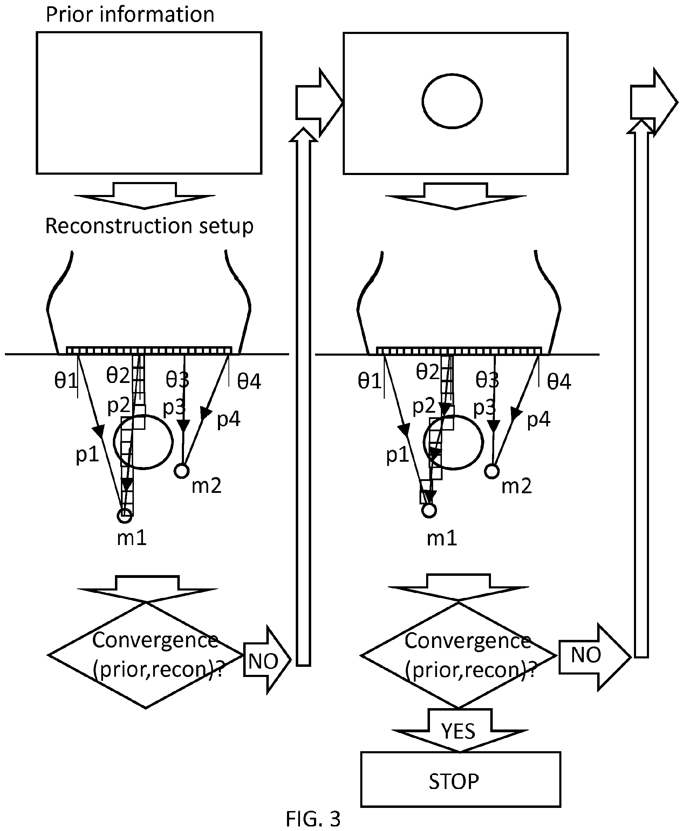

Physical wave propagation trajectories can be incorporated in a straight forward way to the Spatial Domain Regularization (SDR). An example is shown in Figure 3. In a first iteration, a homogeneous background is assumed as prior information and straight ray tracing is used to calculate a speed of sound image. This first image can be used in a second iteration to compute more realistic ultrasound propagation paths in order to improve the beamforming and reduce clutter in the speckle tracking. Moreover, the prior information can be introduced into the spatial regularization, allowing for adjusting the wave propagation paths to describe the actual wave trajectories under consideration of wave refraction effects. This process can be iteratively repeated until a convergence is obtained. Moreover, the full wave equation can be simulated for the prior configuration and a time surface can be computed for each tissue position, from which the gradients determine the possible ray trajectories at all points. It is also possible (time reversal physics) to reverse the wave propagation of received signals through a simulation model - with or without prior inclusion information-, to achieve convergences at originating scatter sources. Overall all these strategies can be used to improve the image contrast and resolution and the speckle tracking accuracy.

**Solving reconstruction problem, inversion of sound-speed matrix**

Finding $\sigma$, which contains the slowness $\sigma_c$ values per cell c, is the inverse problem:

$$\Delta t = L\sigma \qquad\qquad Eq.\ S.1$$

The resulting matrix $\sigma$ hence represents the speed of sound distribution across the cells c, i.e. for the virtual cells the tissue T in the subject plane is divided into, and in particular the cells c that are affected by an inclusion m, see e.g. Fig. 1, given that the speed of sound in such cells is different to the speed of sound in cells that cover non-tumorous tissue.

It is preferred, that for identifying a cell c in the plane, Cartesian coordinates x and y are used, preferably in an orientation with y normal to the transducer surface in the center, also referred to as vertical direction, and x orthogonal to that, also referred to as horizontal direction. The indices i and j are then respectively used to enumerate cells in x and y directions. For 2D matrix array transducers, trivially a 3rd axis z orthogonal to the other two axes can be introduced in order to generate a 3D cell grid for reconstructions in 3D domain.

In a preferred implementation, the quantitative speed of sound values are directly calculated from the slowness values as:

$$v(x,y) = \sigma(x,y)^{-1}$$

In another preferred implementation, both the delays $\Delta t_p$ and slowness increments $\sigma_c$ , as written in equation S.1, represent perturbations caused by inclusions with respect to homogeneous tissue, that is, a tissue model in which no inclusions are present.

Preferably, a pre-step is then used to estimate the average speed of sound $v_B$ out of the measured time delay matrix. $t_p$ then corresponds to the delay residuals after subtracting from $t_p$ the delays caused by the homogeneous tissue, that is,

$$\Delta t_p = t_p - \sum_{c=1}^{C} l_{p,c} / v_B \qquad\qquad \text{Eq. S.2}$$

Quantitative speed of sound images v[m,s] are calculated from $v_B$ and the slowness increments $\sigma$ [s/m] for an individual cell c addressed by coordinates x and y in the plane

$$v(x,y) = v_B (1 + \sigma(x,y))^{-1} \qquad\qquad \text{Eq. S.3}$$

A first estimate of $v_B$ is obtained from the known tissue type (for soft tissue the nominal value is 1540 m/s). Several global optimization methods are known in the literature to estimate $v_B$ to an accuracy of approximately 1%, for instance, by iteratively adjusting $v_B$ until the smearing of speckle patterns is minimized. Also the time measurements obtained for certain diversity configurations will show systematic baseline patterns if the wrong $v_B$ is used. Analysis of such patterns can be used to further refine the value of $v_B$. An example is shown with Eq. E2.

Therefore, it is desired to solve an incomplete reconstruction problem according to equation (Eq. S.2), which is inherently ill-posed. This means that the corresponding mathematical equations cannot be solved uniquely. Several potential solutions for the speed of sound matrix $\sigma$, or more general for the ultrasound parameter matrix, are possible. However, in solving equation (Eq. S.2) it is preferred and desired to find the solution amongst the set of possible solutions that provides the best geometric delineation of inclusions, and the best accuracy for the sound-speed values in the inclusions. However, the set of possible solutions is not to be determined: It is sufficient to determine the solution out of the set of possible solutions without the need to know these other possible solutions.

Preferably, this optimization approach is implemented by:

$$\hat{\sigma} = \arg \min_{\sigma} \left\{ \| \Delta t - L\sigma \|_2 \right\} \qquad\qquad \text{Eq. S.4}$$

wherein it is determined, for which specific speed of sound values $\hat{\sigma}$ out of the possible speed of sound values $\sigma$ the error function $\Delta t$-L*$\sigma$, and preferably the second norm thereof, is minimized. Nevertheless, any norm can be used for this cost term, such as 1-norm L1. However, in some scenarios the solution for $\sigma$ in this numerically solved problem still may result in low image quality, where low resolution in vertical direction can be observed, while artifacts may impede the identification and segmentation of tumors.

Therefore, it is preferred that mathematical regularization is introduced to obtain numerically bounded solutions, which allow for satisfactory reconstructions of the position and geometry of one or more tumorous inclusions in a homogeneous tissue background.

In a first embodiment, a regularizing assumption is introduced for the smoothness of the SoS-image according to:

$$\hat{\sigma}_{TV} = \arg \min_{\sigma} \left\{ \| \Delta t - L\sigma \|_2 + \lambda \| D\sigma \|_n \right\} \qquad\qquad \text{Eq. S.5}$$

Accordingly, not only the error function $\Delta t\text{-}L^*\sigma$ is minimized but a sum of the error function and an additional term $D^*\sigma$. D is a gradient matrix introducing which cells are adjacent to each other, and, correspondingly, $D^*\sigma$ denotes the gradient of the speed-of-sound $\sigma$ of adjacent cells in the plane. The usage of the term $D^*\sigma$ is based on the insight that desired solutions of equation Eq. S.2 show one or more closed inclusion geometries in a homogeneous tissue background. Hence, out of the set of possible SoS values solving the equation Eq. S.2, those are selected, that at least in combination with minimizing the error function with piece-wise constant cell values with sudden transitions where necessary.

However, in another embodiment, D can be any other related property, such as curvature matrix (to regularize 2nd order derivatives), DFT/DCT to regularize frequency components, or any wavelet transform, etc. Hence, the ultrasound parameter values can be dependent on "other linear combinations" D, such as curvature, discrete Fourier/cosine transform, wavelet transform, of the ultrasound parameter values, and thus their derivatives.

In a preferred embodiment, $\|\mathbf{D}\sigma\|_n$ minimizes a sum of horizontal and vertical gradients of the reconstructed image, and $\lambda$ is a constant.

The norm n of the smoothness term $D^*\sigma$ critically influences the reconstruction results. For example, if the L2-norm (n=2), which is defined for an arbitrary vector $x_q$ as $\|\mathbf{x}\|_2 = \Sigma_q(x_q)^2$, is applied to $D^*\sigma$, a closed linear solution (Tikhonov regularization) of equation Eq. S.5 can be found, but smooth gradients are favored with respect to sharp gradients. Large jumps in the SoS values of adjacent cells, which may contain different tissue, are penalized unnecessarily with the L2-norm, creating unrealistically smoothed results.

However, if the L1-norm n=1 is used $\|\mathbf{x}\|_1 = \Sigma_q|x_q|$, which in the context of regularization is also referred to as total variation (TV) regularization or compressive sensing, sharp and smooth gradients are equally weighted, which leads to the reconstruction of piecewise homogeneous regions. With n=1, equation Eq. S. 5 becomes a convex problem, in particular a Second Order Cone Programming problem, which preferably is iteratively solved, with optimization methods such as the Interior Point Method and Alternating Directions Method of Multipliers (ADMM).

According to various embodiments of the present invention, the regularization term that preferably contributes to the optimization can be calculated by one norm which shows TV behavior, such as L1-norm, see Eq. S.6, or L2,1-norms, see Eq S.7:

$$\|\mathbf{D}\sigma\|_1 = \sum_{i,j} |\sigma_{i+1,j} - \sigma_{i,j}| + |\sigma_{i,j+1} - \sigma_{i,j}| \qquad \text{Eq. S.6}$$

$$\|\mathbf{D}\sigma\|_{2,1} = \sum_{i,j} \sqrt{|\sigma_{i+1,j} - \sigma_{i,j}|^2 + |\sigma_{i,j+1} - \sigma_{i,j}|^2} \qquad \text{Eq. S.7}$$

Generally, in such grid-like organization of cells in the plane, where cells are arranged next to each other in rows in the horizontal direction as such forming columns of cells in the vertical direction, each cell has at least one neighbor in the horizontal direction and at least one neighbor in the vertical direction. Hence, such regularization term introduces directional gradients, and specifically gradients along the x-axis and another gradient along the y-axis. The indices i and j of each cell c refers to a position along the x and the y-axis respectively. The resulting SoS based image successfully filters out limited-angle artifacts and delineates closed inclusion geometries. Accordingly, the amount of information available in each angular direction is incorporated into the smoothness reconstruction.

As laid out above, missing angular orientations owed to the non-360° setup of the transducer lead to distortion in the reconstructed SoS image. However, it is known up-front which angular orientations are available for each cell, since the wave propagation / ray paths are defined by the hand-held apparatus, apart from small perturbations introduced by tumorous inclusions. Hence, it is very preferred to weight the SoS gradient contributions in different angular directions, and preferably according to the availability and / or impact of ray information in each of these directions. The resulting regularization may be referred to as "Anisotropically Weighted Spatial Regularization". In a specific embodiment, this concept is combined with the total variation approach and is referred to, in the following, as "Anisotropically Weighted Total Variation" (AWTV).

Hence, in a most basic form for two directions such as the orthogonal directions x and y referred to above, a constant $\kappa$ is introduced in the regularization term as weight, which balances horizontal and vertical gradients according to the available ray information in each direction:

$$\|\mathbf{D}\sigma\|_{AWTV} = \sum_{i,j} \kappa |\sigma_{i+1,j} - \sigma_{i,j}| + (1-\kappa)|\sigma_{i,j+1} - \sigma_{i,j}| \qquad \text{Eq. S. 8}$$

This can similarly be achieved for equation S.7 by weighting axial components differently by parameter $\kappa$. Note

that non-axis-aligned weighting can also be achieved by projecting derivative components in equations S.6 and S.7 onto tensors, although we herein prefer axis-aligned weighting. The weight $\kappa$ can be tuned for each individual cell c. However, in a preferred embodiment, a single value $\kappa$ is defined for the full image, i.e. the same weight $\kappa$ is applied to all gradients of the one axis while the weight 1-$\kappa$ is applied to all gradients of the other axis y. Under the assumption that $\kappa \neq 0.5$, the gradients along one of the directions / axis are emphasized over the gradients along the other direction / axis. In a very preferred embodiment, $\kappa$=0.9.

In another embodiment, the gradient directions used in the regularization is not limited to orthogonal directions. More than two gradient directions can be introduced in the spatial regularization term, which then may be referred to as "Multi-Angle AWTV" (MA-AWTV):

$$\left\| \mathbf{D\sigma} \right\|_{MA-AWTV} = \sum_{i,j} \sum_{\alpha=\{\alpha_1,\alpha_2 \mathrm{K}\, \alpha_N\}} \kappa_\alpha \left| D_\alpha \mathbf{\sigma} \right| \qquad \text{Eq. S.9}$$

where $D_\alpha \sigma = \mathbf{D}\sigma \cdot \mathbf{e}_\alpha$ is the directional derivative along the unit vector with inclination $\alpha$.

Given the maximum available angle $\theta_{\max}$ the gradient directions $\alpha$ for a total of $N_\alpha$ different directions are preferably chosen as follows:

$$\{\alpha\} = \{\phi_i, 180° - \phi_i\}, \quad \phi_i \in \left\{ 0, \theta_{\max} \frac{1}{N_\alpha/2 - 1}, \theta_{\max} \frac{2}{N_\alpha/2 - 1}, \mathrm{K}, \theta_{\max} \right\} \text{Eq S.10}$$

and the weights $\kappa_\alpha$ are preferably calculated with the following algorithm:

  1) Initialize weights $\kappa_\alpha = \{0\}$;
  2) For each wave path p crossing cell c:

    a. calculate the inclination of the wave path $\theta$,
    b. Increase the weight $\kappa_\alpha$ of the closest gradient direction $\{\alpha\}$ with respect to $\phi$ by the geometric overlap of path p with cell c. If $\theta > \theta_{max}$, do not increase $\kappa_\alpha$;

  3) Average $\{\kappa_\alpha\}$ over all cells.

Note that not including the directions outside the imaging angular span [$-\theta_{max}, \theta_{max}$] effectively assigns them no regularization. This avoids over-constraining directions, for which no boundary information is available, which reduces image artifacts. For conventional medical ultrasound arrays, $\theta > 30°$ leads to strong grating lobes and cluttering and are typically not used.

For plane wave emissions as illustrated in Fig. 2a, the maximum angle $\theta_{\max}$ is obtained as the maximum plane wave inclination. For other path/diversity configurations a different subset of angles may provide better results.

If step 3 is omitted, cell-specific $\kappa_\alpha$ values can also be used. In a preferred embodiment, three gradient directions are used, preferably: [$0, \theta_{\max}, -\theta_{\max}$], wherein 0° is defined as first direction y along the y-axis, i.e. orthogonal to the second direction along the x-axis defined by the longitudinal extension of the transducer.

In a preferred embodiment, equation S.5 specifically is embodied as:

$$\hat{\mathbf{\sigma}}_{AWTV} = \arg\min_{\sigma} \left\{ \left\| \Delta\mathbf{t} - \mathbf{L\sigma} \right\|_2 + \lambda \sum_{i,j} \sqrt{\left[ \kappa \left| \sigma_{i+1,j} - \sigma_{i,j} \right| \right]^2 + \left[ (1-\kappa) \left| \sigma_{i,j+1} - \sigma_{i,j} \right| \right]^2} \right\}$$

$$\text{Eq. S.11}$$

In a different embodiment, equation S.5 specifically is embodied as:

$$\hat{\mathbf{\sigma}}_{AWTV} = \arg\min_{\sigma} \left\{ \left\| \Delta\mathbf{t} - \mathbf{L\sigma} \right\|_1 + \lambda \sum_{i,j} \kappa \left| \sigma_{i+1,j} - \sigma_{i,j} \right| + (1-\kappa) \left| \sigma_{i,j+1} - \sigma_{i,j} \right| \right\}$$

$$\text{Eq. S.12}$$

And in a further embodiment, equation S.5 specifically is embodied as:

$$\hat{\boldsymbol{\sigma}}_{MA-AWTV} = \arg \min_{\sigma} \left\{ \left\| \Delta \mathbf{t} - \mathbf{L}\boldsymbol{\sigma} \right\|_1 + \lambda \sum_{i,j} \sum_{\alpha=\{\alpha_1,\alpha_2\mathrm{K}\,\alpha_N\}} \kappa_\alpha \left| D_\alpha \boldsymbol{\sigma} \right| \right\}$$

Eq. S.13

To keep the same regularization constant for equation S.11 and equation S.12, the weights are preferably normalized such that $\Sigma_\alpha \kappa_\alpha$ = 1.

It is also possible to use L2,1-norm of Eq. S.7 for the error function term, $\|\Delta\mathbf{t}\text{-}\mathbf{L}\sigma\|_{2,1}$, or any other norm that shows TV behavior.

In a preferred embodiment, the constant λ, also referred to as regularization constant, is set depending on one or more of an image resolution, aspect ratio, the number of time measurements M. The image resolution may be given by parameter h, which denotes the height of a cell, and preferably also the width of a cell in case of square cells. An empirical formula is provided below in Eq. E.1, which may need to be adjusted for different path diversity configurations.

In a preferred embodiment, the ultrasound parameter values are determined for several emitted ultrasound frequencies allowing to reconstruct frequency-dependence of such parameter. The measurement preferably would be repeated while setting the emitter frequency to different values in the ultrasound machine. This may allow for nonlinear SoS and attenuation reconstruction. Then, different reconstructed parameters may, e.g. in their rate of change per frequency, reveal information.

In a preferred embodiment, for a specific line of cells, and most preferably for the lowest or the highest row of cells in the horizontal direction x no regularization is applied. Hence, the values for those cells are found based solely on the error function according to equation S.4 when looking for the best speed of sound values for the cells of this row. By such means baseline artifacts can be released: Small DC components in the slowness distributions σ may lead to staircase artifacts in the vertical direction of the images reconstructed with equation S.11 or equation S.12. These artifacts can be minimized by defining a release line in the image, i.e. a row of cells, for which no smoothness regularization is applied. Typically this is performed for the lowest horizontal line (j = 1). The release line accumulates DC components in σ, which are then homogeneously distributed over the image. Equation S.11 is then rewritten as:

$$\mathbf{v} = \arg \min_{\sigma} \left\{ \left\| \Delta \mathbf{t} - \mathbf{L}\mathbf{v} \right\|_1 + \lambda \sum_{i=1,j=2}^{I,J} \kappa \left| \sigma_{i+1,j} - \sigma_{i,j} \right| + (1-\kappa) \left| \sigma_{i,j+1} - \sigma_{i,j} \right| \right\}$$

$$\hat{\boldsymbol{\sigma}}_{AWTV} = \mathbf{v} + (IJ)^{-1} \sum_{i=1}^{I} \sigma_{i,1}$$

Eq. S.14

Moreover, in the edges of the reconstructed image the gradients are not defined. Preferably, $\sigma_{I+1,j}$ =0, $\sigma_{j,J+1}$ =0 is set as boundary condition, which leads to a minimization of the edge slowness values, and provides a good stability in the reconstructions.

In an embodiment, prior information may be available with respect to the tissue to be examined. In such scenario, constant SoS values may be assigned for some regions of the reconstructed image. For instance, given breast tissue, a constant sound speed value each may be assigned one or more of cystic regions or fat layers. Prior information preferably referring to a region in the tissue may be introduced with the following preferred algorithm:

1) Error function $\|\Delta\mathbf{t}\text{-}\mathbf{L}\sigma\|_m$

    a) Group all σ values belonging to the same region prior information is available for region into a single value;
    b) Sum all columns of **L** corresponding to the grouped σ values;

2) Regularization term $\|\mathbf{D}\sigma\|_n$

    a) Group all σ values belonging to the same region prior information is available for into a single value;
    b) Sum all columns of **D** corresponding to the grouped σ values.

In this embodiment, a region comprising multiple cells in the plane is treated uniformly and is assigned the known speed of sound value. Such a grouped σ region shows longer associated relative path lengths $l_{p,c}$ in **L**. Consequently, an error weighting of the grouped region preferably is proportional to their surface.

Since the gradient matrix **D** preferably contains differences of the form [+1, -1] for adjacent cells, regularization constraints corresponding to grouped σ values will vanish. However, the edges of the prior known regions preferably will preserve the regularization constraints.

Total variation, as L1 norm used in embodiments of the reconstruction of the image, in particular performs well in reconstructing piecewise constant image regions such as inclusions, as typical for tumors and their surroundings. However, in some scenarios, it may be required to reconstruct smooth SoS regions. In these cases, the above total variation may show staircase artifacts. A possibility to alleviate these effects is to consider higher order differences in the smoothness regularization. A particular example is Total Generalized Variation:

$$\|\mathbf{D}\boldsymbol{\sigma}\|_{TGV} = \min_{\mathbf{v}}\left\{\|\mathbf{D}\boldsymbol{\sigma} - \mathbf{v}\|_1 + \|\mathbf{D}\mathbf{v} - \mathbf{D}\mathbf{v}^T\|_1\right\} \qquad \text{Eq. S. 15}$$

which balances between the first and second derivatives of the function. Accordingly, in another embodiment, the processor is configured to determine the speed of sound values by minimizing according to the following function:

$$\|\mathbf{D}\boldsymbol{\sigma}\|_{TGV-AWTV} = \min_{\mathbf{v}}\left\{\sum_{i,j}\left|\kappa(\sigma_{i+1,j} - \sigma_{i,j}) - v_1\right| + \left|(1-\kappa)(\sigma_{i,j+1} - \sigma_{i,j}) - v_2\right| + \|\mathbf{D}\mathbf{v} - \mathbf{D}\mathbf{v}^T\|_1\right\}$$
$$\text{Eq. S. 16}$$

Other examples include bilateral filter, guided filters and curvature filters, among others, which accommodate specific curvatures/smoothness (piecewise linear, piecewise smooth, Gaussian).

**Extension to other acoustic parameters**

Spatial Domain Reconstruction refers here to the reconstruction of parameters that characterize the transmission of ultrasound through tissue structures. It does not refer to beamforming methods to generate images of tissue reflectivity patterns (conventional B-mode images).

The ultrasound parameter that is determined can be one of:

- speed of (ultra) sound
- acoustic attenuation
- frequency dependent acoustic quantities, e.g. frequency dependent SoS and attenuation
- speed of sound dispersion

In one embodiment, the tomographic image reconstruction is based on acoustic attenuation. The acoustic attenuation $\alpha(dB/cm)$ describes the loss of signal amplitude due to absorption and scattering in tissue. Attenuation measurements can be performed as follows with any embodiments of the invention.

For instance, in the registration method described in Eq T.1, $\sigma_x[m]$ and $\sigma_y[m]$ represent cumulative energy values for the measurement point m for two different wave propagation paths. These, for instance, can be computed from signal envelopes, signal peaks, or similar methods known to those skilled in the art. In one embodiment, speed-of-sound reconstructions may be used to compensate for aberrations between different paths to better align signals for more accurate comparative energy measurements. Relative amplitude measurements can be expressed in linear scale as:

$$a[m] = \sigma_y[m]/\sigma_x[m] \qquad \text{Eq. A.1}$$

The amplitude measurements are a function of the speckle pattern reflectivity and the acoustic attenuation. The speckle pattern reflectivity can be modeled in function of the path direction, and removed from the relative amplitude measurements. The remaining amplitude variations are due to acoustic attenuation $\Delta a_m$. For instance, if isotropic reflectivity patterns are assumed, then $a_m = \Delta a_m$. Expressing then $\Delta a_m$ in logarithmic units a formally equivalent relation to Eq. D.4 in terms of acoustic attenuation $\alpha$ [dB/m] at tissue cells c is obtained:

$$20\log(\Delta a_m) = \sum_{c=1}^{C} l_{m,c}\alpha_c \qquad \text{Eq A.2}$$

Eq. A.2 can be solved with all the methods described in the invention for Eq. D.4. All image reconstruction methods described in connection with speed of sound can be applied.

In another embodiment, the tomographic image reconstruction is based on frequency-dependent acoustic quantities. For instance, given the registration method described in Eq T.1, the power spectral densities at the measured tissue position m for both paths $S_x[m,f]$ and $S_y[m,f]$ from the sample sets $y\lfloor m + n_{opt} + k \rfloor$ and $x[m+k]$, with $k=[-W/2,W/2]$ defining the correlation block size. $S_x(m,f)$ and $S_y(m,f)$ are computed in a straight forward way with spectral estimation methods well known in the art, e.g., Bartlett's method, least-squares spectral analysis, wavelet analysis or short-time Fourier transform. In this process, windowing functions can be applied to the samples in $y\lfloor m + n_{opt} + k \rfloor$ and $x[m + k]$ to reduce edge discontinuities, e.g. Hanning, Gaussian, etc. Then, frequency-dependent relative amplitude measurements are calculated:

$$a[m,f] = S_y[m,f]/S_x[m,f] \qquad \text{Eq. A.3}$$

Applying then Eq A.2, the acoustic attenuation can be solved in function of the frequency $\alpha_c(f)$.

A similar method can be applied to compute frequency-dependent speed of sound values by analyzing the phase information contained in $y\lfloor m + n_{opt} + k \rfloor$ and $x[m + k]$. First, $y\lfloor m + n_{opt} + k \rfloor$ and $x[m + k]$ are expressed in the frequency domain $f$, for instance with a Fourier, cosine or wavelet transform. The phase differences $\phi_y(m,f)$-$\phi_x(m,f)$ in function of the frequency $f$ are then directly associated to frequency-dependent relative time measurements $\tau_m(f)$:

$$\tau_m(f) = \lfloor \phi_y(m,f) - \phi_x(m,f) \rfloor / (-2\pi f) \qquad \text{Eq. A.4}$$

Given $\tau_m(f)$, all image reconstruction methods described in connection to speed-of-sound can be applied to invert the frequency-dependent speed-of-sound distribution $\sigma_c(f)$ for each tissue cell c. The speed of sound dispersion $x_c(f)$ can then be obtained at each cell as follows from both $\sigma_c(f)$ and the average speed of sound value $v_B$, the latter of which can be measured with one of the aforementioned methods.

$$v_B(c,f) = v_B\left(1 + x_c(f)\right) = \left(v_B^{-1} + \sigma_c(f)\right)^{-1} \qquad \text{Eq. A.5}$$

**Reconstructions with Multiple Measurements**

Multiple ultrasound data collected from several observations can also be combined in the reconstruction equation above to solve altogether at once, to increase accuracy and robustness. Expected consistency of additive displacements across several observation frames may also be used to boost accuracy and resolve ambiguities by removing outliers in displacement estimations similarly to registration rectification methods in [3].

**A possible embodiment of measurement setup**

In such embodiment, the ultrasound transducer comprises at least an element for emitting ultrasound, preferably at some frequency in a range between 1 MHz and 40 MHz, and more preferably in a range between 3 MHz and 14 MHz. The ultrasound transducer preferably converts electrical signals into ultrasound waves, e.g. by means of a piezoelectric converter as such element. In a very preferred embodiment, the ultrasound transducer also comprises at least one receiver element, and preferably more, for receiving ultrasound waves, and in particular for receiving reflected ultrasound waves as will be explained below, and for converting the received ultrasound waves into electrical signals.

For this purpose, the ultrasound transducer comprises a set of emitter elements and a set of receiver elements. While the elements of the sets may be different elements such that emitter elements only are capable of emitting ultrasound while receiver elements only are capable of receiving ultrasound, in a different embodiment a single transducer element may be configured to emit and receive ultrasound. Such transducer element is referred to act as emitter element and as receiver element respectively. Each set preferably comprises two or more elements, and preferably more than hundred elements.

Preferably, a combination of an emitter element and a receiver element - also referred to as pair - is operated at the same time, i.e. the processor triggers the respective emitter element to emit an ultrasound wave, while the receiver element receives the emitted and reflected ultrasound wave with a certain delay.

At the receiver element, the received reflected ultrasound wave is converted into an electrical signal over time, also referred to as radio frequency (RF) trace.

Given that various emitter - receiver element combinations are triggered sequentially by the processor, it is preferred that for each combination the corresponding RF trace is recorded. Preferably, all possible emitter element - receiver element combinations are triggered and define the set of combinations. However, in a different embodiment, only a selection out of all possible combinations is defined in the set of combinations.

In a preferred embodiment, a single transducer with N transducer elements is applied, and a "multi-static matrix", with RF traces, and / or corresponding time of flight values for all possible $N \times N$ emitter element - receiver element combinations is recorded. It is then preferred, that for identifying a certain path p, an index for the transducer emitter element e and the transducer receiver element r are used, so that the time of flight tp and te,r are equivalent. It is possible to acquire the "multi-static matrix" in real time, for instance, by simultaneously recording the RF traces of multiple receiver elements.

Other variations are possible: For instance, several adjacent emitter elements may be fired simultaneously or with an incremental time-delay, generating a so-called "plane-wave" emission, to increase an acoustic intensity level coupled into the measured tissue, and/or the RF traces of several adjacent transducers may be averaged or combined in any form to reduce noise. Hence, the preferred starting point to the image reconstruction is a set of digitized RF traces acquired by individual receiver elements upon specific emitter firings, hence, each corresponding to an emitter element - receiver element position pair.

In another embodiment, the apparatus may include a matrix transducer with a two-dimensional array of transducer elements, which allows the processing unit to reconstruct three-dimensional images, by combining emitter - receiver pair information in different planes. In a different embodiment, the two-dimensional hand-held apparatus can be used multiple times, each in a different plane, in order to generate a three-dimensional image stack. The here outlined hand-held apparatus can also be incorporated to an automated scanning system that provides three-dimensional image stack, but sequentially moving along multiple planes, which sequential movement is automatically controlled.

In a different embodiment, the two-dimensional hand-held apparatus can be used multiple times, each in a different plane, in order to generate a three-dimensional image stack. The here outlined hand-held apparatus can also be incorporated to an automated scanning system that provides three-dimensional image stack, but sequentially moving along multiple planes, which sequential movement is automatically controlled.

In a preferred embodiment, the transducer has a linear array of transducer elements, and hence, a flat, longitudinal extension along these elements. However, other geometries of the transducer are possible, for instance, convex implementations. Preferable, the geometric paths between transducer pairs and specific internal tissue positions can be defined for such other geometries, which in general is possible for any arbitrary geometry. For this purpose, ray tracing equations or more advanced full wave simulation approaches, e.g. finite-difference time-domain simulations, can be applied.

**Applicability scenarios**

It is preferred that the apparatus is used in Ultrasound Computed Tomography (USCT) in the medical domain to detect tumorous inclusions in breast tissue, which may not be visible in conventional B-mode images or may be visible but may not be diagnosed or categorized in B-mode images alone.

Preferably, the ultrasound parameter that is determined per cell can be one of speed of (ultra)sound, acoustic attenuation, frequency dependent acoustic quantities, speed of sound dispersion. Although the previous and following embodiments are mostly referred to the speed of sound determined as ultrasound parameter, it is understood that in any of the following embodiments, the speed of sound may be replaced by acoustic attenuation as relevant ultrasound parameter, or any of the other parameters as listed.

The measured ultrasound parameters can be in turn combined to estimate other tissue properties, such as for instance the tissue temperature (e.g., during an ablation treatment), or the mass density, or in general any property of healthy or diseased tissue, which correlates with the measured ultrasound parameters. Repeated ultrasound measurements can be used to monitor tissue changes in time.

The measured ultrasound parameters can as well be determined in function of an external perturbation applied to the tissue, such as a mechanical excitation (for instance, a pre-compression or a vibration field, such as vocal fremitus), or a temperature field (for instance, during an ablation treatment), among others.

A standard ultrasound transducer can be employed, which is known e.g. from conventional B-mode scanning, in contrast to customized and costly transducer mechanisms of the known systems, which then also allows a clinician to simultaneously use this transducer for conventional clinical B-mode imaging, by simply using different processing pipelines for the recorded data.

The presented invention provides a low-cost hand-held alternative to state-of-the-art high-end ultrasound tomography systems. Conventional B-mode systems can be used for USCT with the same hardware plus dedicated software. The present invention can be used as an add-on to conventional B-mode ultrasound equipment, particularly for breast cancer detection. The invention may allow for the detection of other anomalies of the subject tissue such as lesion / fibroadenoma / cysts, also giving information about size and / or depth and / or location.

Apart from breast scanning, a broad range of other applications and targets may be envisaged, medical imaging for other organs and tissue structures which can be measured with conventional ultrasound waves such as musculoskeletal system, liver, kidney, spleen, prostate, brain, thyroid, and in general for non-destructive testing of materials, biological or non-biological. The hardware (for instance, transducer geometry, pulse frequency, duration and signal level) and software (regularization parameters, amplitude and displacement tracking algorithms) can be optimized for each application scenario.

**Implementation examples**

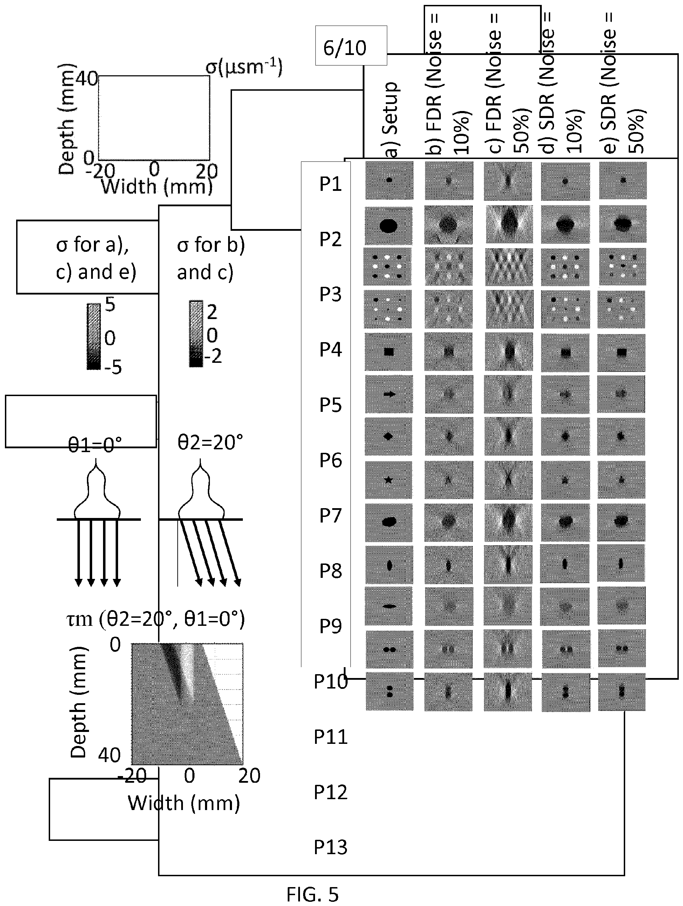

FIGURE 5 illustrates in columns a) ten different examples of artificial inclusions (black and white) in a homogeneous tissue (grey). Black colors correspond to high speed of sound values, whereas white color corresponds to low speed of sound values. Columns b) to e) show images based on simulation results of a virtual transducer extending at the top line of each sample. Relative time measurements $\tau_m$ are simulated at each tissue cell coordinate c by comparing plane wavefronts at angles $\Theta1$ = -20° and 20° with respect to $\Theta2$ = 0°. This follows the spatial diversity generation strategy illustrated in Fig. 2a. The $\tau_m$ values are directly generated with a ray tracing algorithm, so that no uncertainties due to texture matching or unaccounted wave propagation paths are present. Random noise is then added to the $\tau_m$ matrix, following a Gaussian distribution with zero mean and a standard deviation expressed as percentage (10% or 50%) of the peak $\tau_m$ value for each example. An example of $\tau_m$ is plotted for tissue model P1 and noise level = 10%.

The Spatial Domain Reconstuction (SDR) obtained with an embodiment of the proposed invention are compared to reconstructions obtained with the same setup for the Frequency Domain Reconstruction (FDR) method known from previous art WO 2015/091519. Specifically, for SDR the "multi-angle anisotropically weighted total variation (MA-AWTV)" with three gradient directions is used Eq. S.13. The regularization parameters are adjusted in both FDR and SDR cases so that the best possible image quality is obtained (above the noise level with best vertical resolution). The same regularization parameter $\lambda$=0.0125 is used for all SDR examples. The empty regions of $\tau_m$ due to the non-adjacent plane wave regions (Fig. 2b, dashed regions) are not included in the reconstruction for SDR, and zero valued for FDR.

The FDR images are of relatively lower contrast already at moderate noise levels of 10%, such that the inclusion geometries P6 and P8 and P11 cannot be resolved. For noise levels of 50%, the inclusion geometries P3, P4, P5, P6, P7, P8, P9 cannot be resolved. Two kinds of image distortion, which were previously reported by WO 2015/091519, or Jaeger et al. 2015 are observed, which are common to limited-angle computed tomography algorithms: a) Resolution loss along missing angular directions: for instance, since ray paths parallel to the transducer surface are missing, the reconstruction precision is significantly lower in the vertical direction, b) strong streaking artifacts: these are the consequence of the regularization in the frequency domain, and probably due to the steep elimination of certain frequency components from the image spectrum, c) artifacts are also observed at the bottom of P2 due to the zero-filled $\tau_m$ regions. The speed-of-sound values reconstructed with FDR are also significantly reduced with respect to the defined setup values.

On the contrary, the spatial regularization applied by the SDR method presently described achieves piecewise delineation of homogeneous inclusions, effectively filtering out noise from the images. Both reconstructions at noise level 10% and 50% show consistent results, with minor image degradation. Remaining artifacts include the disappearance of one of the inclusions of P4 for noise = 50%, corresponding to a corner region where only few $\tau_m$ measurements are available.

Therefore, given the same $\tau_m$ data, the proposed SDR method achieves a significant improvement of inclusion delineation for moderate noise scenarios with respect to prior art. Quantitative speed of sound images are obtained, which closely match the defined setup values. Therefore, a quantitative imaging biomarker can be obtained from this method for tissue differentiation and diagnosis.

Fig. 6 and Fig. 7 provide examples of the clinical applicability of embodiments of the present invention to tumor delineation and differentiation. In-vivo data were recorded at the University Hospital of Zurich for a female patient showing a cancerous lesion - which biopsy revealed as invasive ductal carcinoma (Fig. 6), and a second female patient showing a benign lesion - confirmed to be a fibroadenoma (Fig. 7). Conventional ultrasound B-mode images are provided for both lesions, both appearing as a dark region followed by a shadow region, which do not show a delineation and differentiation of the lesions in terms of gray level contrast. Multi-static data A(t, tx, rx) was acquired based on a commercial FDA-approved SonixTouch research ultrasound machine, from Ultrasonix Medical Corporation, Richmond, BC, Canada. The ultrasound transducer is here a commercial linear array (L14-5, Ultrasonix). It comprises a total of N=128 transducer elements, with a pitch between elements of 300 $\mu$m, an element elevation of 7 mm, and a total aperture of 38 mm. A multichannel data acquisition board (SonixDaq, Ultrasonix) collects raw pre-beamformed data in parallel with a sampling frequency of fs = 40 MHz and 12 bits per sample. The process is repeated for all transmitter elements, so that a total of $N^2$ time traces were recorded in <0.1 s (about 100 MB). The transducer provides two-dimensional ultrasound imaging in a plane perpendicular to the transducer elements, with the width of the image corresponding to the linear array direction, and the depth corresponding to the perpendicular direction to the transducer elements, along which ultrasound echoes are recorded in function of time. Plane wavefronts were synthesized from the multi-static data, see Eq. P.1, between $\theta$ = -22.5° and 22.5° with 0.5° steps. Beamformed images B(x,y,$\theta$) were generated at each width x (mm) and depth y (mm) coordinate were calculated with Eq. P.2. for each angle $\theta$, by choosing the delays(rx) as:

$$\texttt{delay(rx) = 1/v}_\texttt{B}\texttt{* (x*sin}(\theta)\texttt{+ y*cos}(\theta)\texttt{+}$$

$$\text{sqrt}((x - \text{pitch*rx})^2 + y^2))$$

The displacement tracking (motion estimation) algorithm described in Eq T.1 was applied between beamformed images corresponding to increasing $\theta$ pairs. A texture correlation block of 1x1 mm$^2$ (52x3 pixels$^2$) was used, with a search range of 0.125 us. A one-dimensional correlation search was performed in $y$, using a zero-normalized cross-correlation (ZNCCC) to find the vertical displacement $\Delta y$ in metrs at each point (x, y). $\Delta y$ is calculated from Eq T.2 as:

$$\Delta y = n_\text{opt}\text{*pixel\_size\_y;}$$

where nopt is the displacement in pixels and pixel_size_y is the recorded pixel size in $y$. For a sampling frequency fs of the ultrasound system [s$^{-1}$], pixel_size_y is calculated as:

$$\texttt{pixel\_size\_y = 1/2/fs*v}_\texttt{B}$$

The vertical displacement measurements $\Delta y$ were then converted to time measurements with:

$$\tau_\text{m} = \texttt{n}_\text{opt}\texttt{*/v}_\texttt{B}\texttt{/cos}(\theta)$$

Time measurements $\tau_m$ with correlation coefficients $c_{yx}\left[m,n_{opt}\right] > 0.5$ were discarded from the measurements. The time measurements were cumulated so that time measurements of [$\theta$ = -22.5, -17.5, -12.5, -7.5, -2.5, 2.5, 7.5, 12.5, 17.5, 22.5]° with respect to 0° were input to the reconstruction. Specifically, for SDR the "multi-angle anisotropically weighted total variation (MA-AWTV)" with three gradient (-22.5°, 0°, 22.5°) directions was used.

The path lengths $l_{p,c}$ were calculated with linear interpolation.

The regularization constant was calculated for this configuration as follows:

$$\lambda = \lambda_{ref}\frac{h}{h_{ref}}M\sqrt{\frac{W}{D}} \qquad\qquad \texttt{Eq. E.1}$$

where h is the output pixel size (here 0.5 mm), *href* equals the transducer pitch (300 $\mu$m), D and W are the reconstructed image depth and width, and M is the number of time measurements incorporated considered in the inverse problem Eq. D.4. The reference regularization constant depends on the spatial diversity strategy used and the type of spatial regularization applied. For plane waves and MA-AWTV with L1-norm solution term, it has been empirically found that $\lambda_{ref}$ = 2.1E-7, provides a good performance for a broad range of experimental configurations. Accordingly, in the calculated examples, $\lambda$=0.09.

Prior to the SDR reconstruction, the average speed of sound $v_B$ was adjusted to its actual value as follows: Considering two different plane wave angles $\Theta$1 and $\Theta$2 and an initial estimate of the average speed of sound $\hat{v}_B$, the misadjustment of $v_B$ translates into an increasing time delay with depth coordinate $y$:

$$\tau(y) = y\left(\cos^{-1}\theta_2 - \cos^{-1}\theta_1\right)\left(v_B^{-1} - \hat{v}_B^{-1}\right) \qquad \text{Eq. E.2}$$

from where the actual average speed of sound $v_B$ can be estimate from a linear fit.

Figure 6 and Figure 7 show speed-of-sound images reconstructed with the SDR method. The measurement data $\Delta t$ shows large empty regions corresponding to the tissue regions no displacement information could be extracted. These were observed for instance in image areas not traversed by the plane wavefront, but also in the low echogenicity region observed around the breast lesions. In general, any displacement tracking algorithm may show tracking failures, corresponding to image areas, where speckle information is not sufficient for extracting displacements. The presently suggested SDR reconstruction can handle these empty regions, allowing inputting incomplete tracked frames into the reconstruction.

An FDR requires a complete $\Delta t$ measurement for all the tissue region. The combination of a large part of measurement data missing, together with the noise of the speckle measurements leads to a poor reconstruction, in which the tumorous inclusions cannot be delineated. On the contrary, the SDR reconstruction provides a clear delineation of the malignant tumor region, in agreement with the inclusion visible in the B-mode image. Moreover, the sound-speed imaging allows for a sharp delineation of the tumor area where sound-speed changes occur, which is not possible in the B-mode images, due to the shadowing (increased acoustic attenuation) behind the lesions. A clear differentiation is observed between malign (>5% speed-of-sound contrast) and benign lesions (about 2% speed-of-sound contrast), which allows for quantitative differentiation. These values are in quantitative agreement with prior speed of sound observations obtained with full-angle ultrasound computed tomography (USCT) of the breast. It is remarkable that despite the lack of time measurement information in the malign tumor region of Fig. 6, the tumor can be sharply delineated. As illustrated in Fig. 1, the required reconstruction information is obtained from behind the tumorous lesion, corresponding to paths that have traversed the lesion. The Spatial Domain Regularization (SDR) approach presently presented is capable of capturing irregularly distributed time measurement information and correctly regularizing it for a sharp delineation of speed of sound inclusions.

**Displacement estimation for reflector-less reconstruction**

Displacement estimation preferably is used in elastography, where local displacements are observed in response to some external excitation in order to estimate tissue viscoelasticity. Displacement tracking preferably is used in order to infer apparent displacements due to differences in speed-of-sound (SoS). The accumulation of local SoS along different sound paths cause a physical point in space to appear at slightly different locations. When two beamformed frames from two different viewing angles are compared and the apparent displacement between them are estimated, this represents the difference of cumulative SoS between rays (waves) arriving at that point from those two angular directions.

- For displacement tracking, there exist several techniques, including gradient-based, phase-based, and Doppler-based methods. Displacement tracking methods are typically classified based on the type of signal [RF, in-phase and quadrature (I/Q), envelope, and B-mode] and the domain on which they operate (i.e., time, phase, or frequency).
- Since phase based estimators are limited by a horizon of half ultrasound wavelength, phase unwrapping preferably is also applied in displacement tracking. Peak-based estimators can preferably also be applied.
- To increase precision, up-sampling methods, as well as subsampling using function fitting or pattern matching techniques may also be suggested.
- Although a 1D displacement estimator may be used, multi-dimensional (e.g. 2D) displacement/motion estimation techniques may help leverage complementary information from different axes, and thus resolve estimation ambiguities.
- Elastography often uses the temporal consistency of motion across consecutive image frames in time. Similarly, the consistency of apparent displacements across multiple consecutive plane-wave angles may be used to filter out observation outliers.

The image processing and analysis techniques of optical flow and image registration may also be capable of finding displacements. This then allows for standard techniques, such as parametric (B-spline) or non-parametric displacement estimators, as well as using the consistency of displacement across several frames to increase accuracy and resolve ambiguities by removing outliers in displacement estimations.

The content of the following papers is referred to:

[1] F. Viola and W. Walker, "A comparison of the performance of timedelay estimators in medical ultrasound," IEEE Trans. Ultrason. Ferroelectr. Freq. Control, vol. 50, pp. 392-401, Apr. 2003.

## EP 3 577 491 B1

[2] Reza Zahiri-Azar, Orcun Goksel, and Septimiu E. Salcudean: "Sub-sample Displacement Estimation from Digitized Ultrasound RF Signals Using Multi-Dimensional Polynomial Fitting of the Cross-correlation Function", IEEE Trans Ultrasonics, Ferroelectrics, and Frequency Control 57(11):2403-20, Nov 2010.

[3] Tobias Gass, Gabor Szekely, and Orcun Goksel: "Consistency-Based Rectification of Non-Rigid Registrations", SPIE J Medical Imaging 2(1):014005, May 2015.

**General**

In one embodiment, the ultrasound transducer may comprise a set of transducer elements each containing one emitter element and one receiver element, or a combined emitter and receiver element.

In one embodiment, the emitter elements of the set and the receiver elements of the set, or the transducer elements respectively, are arranged in a straight row. Preferably, the first direction of the gradients of the ultrasound parameters is a direction y orthogonal to a longitudinal extension of the set of emitter elements Tx and the set of receiver elements Rx, or the transducer elements respectively, and the second direction x is orthogonal to the first direction y.

In a different embodiment, the emitter elements of the set and the receiver elements of the set, or the transducer elements respectively, are arranged in a curved line, and preferably along a convex line. Each transducer element, or each emitter element and receiver element, has a principal direction referred to as radiation axis along which the transducer element emits ultrasound. Preferably, the first direction y of the gradients of the ultrasound parameters represents an average of the radiation axes of the set of emitter elements Tx and the set of receiver elements Rx, or the set of the transducer elements respectively. The second direction x is orthogonal to the first direction y.

In a different embodiment, the second direction is defined by a maximum angle $\phi_{max}$ with respect to the first direction, which maximum angle $\phi_{max}$ is defined by a maximum inclination of the ultrasound beam or non-focused ultrasound wavefront transmitted by the set of emitter elements Tx and received by the set of receiver elements Rx, and a third direction preferably is defined by the negative maximum angle ($\phi_{max}$).

In this context, every transducer element may have a defined radiating axis y. However, several transducer elements together (array) can allow angulation of the ultrasound transmission, for instance, by generating plane waves with a defined angle, or by generating a steered ultrasound beam. For a particular measurement scenario, a maximum angle that preferably can be generated is referred to as $\phi_{max}$ which preferably is dependent on physical constraints as side lobes, or design decisions to use just a limited set of angles.

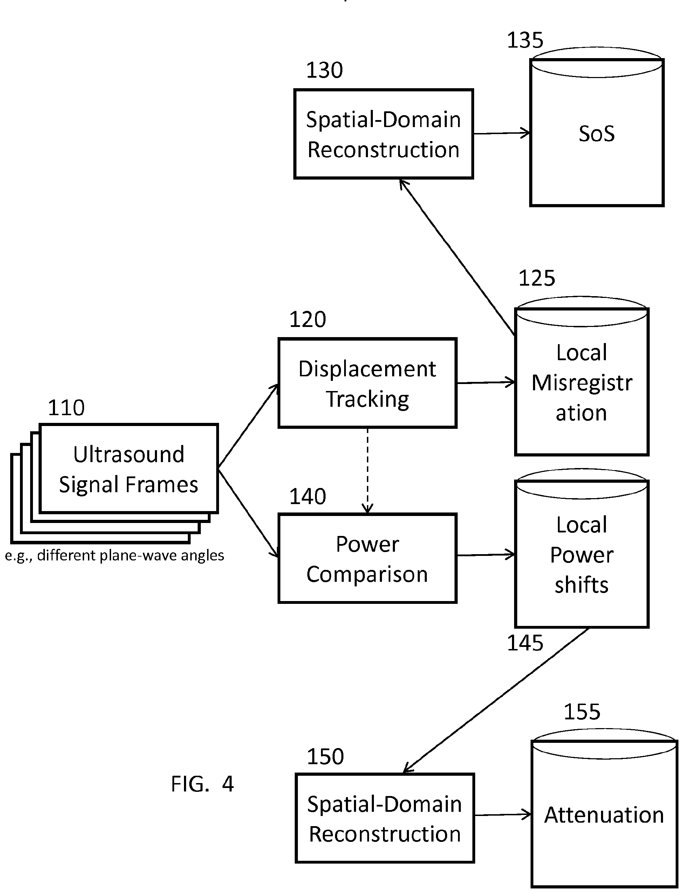

Fig. 4 illustrates a block diagram of a medical ultrasound system according to an embodiment of the present invention, employing two different approaches in generating an ultrasound based tomographic image, and including one or more preferred buildings blocks already addressed in the previous sections. In different embodiments, only one of the approaches of generating an ultrasound based tomographic image is implemented and is applied in a medical ultrasound system.

For both approaches, ultrasound signal frames are generated from the tissue to be investigated in block 110, preferably by means of an ultrasound transducer. Each frame preferably represents an ultrasound wave received by one or more receiver elements of the transducer in response to an ultrasound wave emitted by one or more of the emitter elements of the transducer. Each frame, and hence each measurement preferably is taken from a different plane-wave angle, i.e. by holding the transducer in a different angle relative to the tissue to be investigated, such as is illustrated in Fig. 1, for example.

In the first approach, i.e. a first embodiment, timing of the ultrasound signal frames corresponding to different paths through the tissue / angles of the ultrasound transducer is evaluated. For this purpose, displacement tracking is performed in block 120 for any frames corresponding to different measurement angles, however with a focus on the same location of the tissue. Next, local mis-registration between two or more frames is performed in block 125. Based on such mis-registrations, represented e.g. by differences in the measurements in time, a spatial-domain reconstruction is applied in block 130, which effectively leads to a determination of a speed of sound distribution over the discretized tissue region, see block 135. The speed of sound distribution may then be graphically displayed as ultrasound based tomographic image.

In a second approach, i.e. a second embodiment, the ultrasound parameter to be determined per discretized cell of the tissue is not speed of sound but attenuation, instead. Accordingly, it is not timing information in the received ultrasound signal frames that is evaluated, but it is the amplitude or power of the ultrasound signal frames received, see block 140. Hence, an amplitude or power comparison is performed for any frames corresponding to different measurement angles, however with a focus on the same location of the tissue. Next, instead of local timing mis-registrations, local amplitude / power shifts are determined in block 145 for the respective signal frames. Again, a spatial domain reconstruction is applied in block 150 providing an attenuation distribution over the discretized tissue region, see block 155. The attenuation distribution may then be graphically displayed as an ultrasound-based tomographic image.

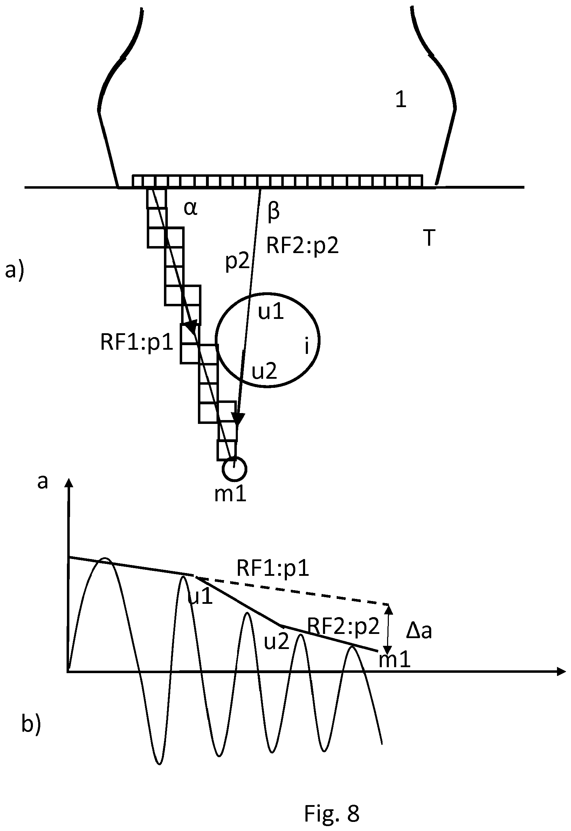

FIG. 8 illustrates a method and a medical ultrasound system according to an embodiment of the present invention. The physical set-up of the medical ultrasound system of FIG. 8a) is identical to the set-up shown in FIG. 1.

However, the ultrasound parameter extracted from the measurements is different: While in Fig. 1, the ultrasound parameter investigated is speed of sound, in FIG. 8, it is attenuation of the ultrasound wave. Accordingly, if put into context with the embodiment of FIG. 4, the embodiment of FIG. 1 may represent a system / an approach including the upper branch of the block diagram of FIG. 4, while the embodiment of FIG. 8 may represent a system including the lower branch of the block diagram of FIG. 4.

In the illustration of Fig. 8a), again, two sample ultrasound waves are directed towards location m1 in the waveplane. In this example, the waves are referred to as RF1 and RF2, wherein wave RF1 takes path p1 inclined by angle $\alpha$ with respect to the row of the elements of the transducer 1, while wave RF2 takes path p2 inclined by angle $\beta$ with respect to the row of elements of the transducer 1. Along path p1, there is no inclusion, while along path p2, inclusion i is traversed between locations u1 and u2.

In Fig. 8b), an envelope of the amplitude or power of the corresponding waves is referred to by RF1 and RF2 their travel through the tissue T. It can be derived, that wave RF1 not passing any inclusion is less damped than wave RF2 passing the inclusion i. A difference in the envelope of the amplitudes of the waves (such as received at the corresponding receiver elements) is referred to as shift in amplitude or power $\Delta a$ in FIG. 8b), comparable to the difference of time of flight values for the embodiment of Fig. 1. Accordingly, instead of a time difference between the time of flight values of two waves travelling different paths through the tissue resulting in the determination of speed of sound values for the individual cells, such as shown in FIG. 1, now a difference between the amplitudes / powers of two waves - and more precisely of the two reflected waves as received by the receiver elements - allows for determining attenuation values for the individual cells according to FIG. 8.

Analogue to equation 5.1, the equation for the attenuation being the relevant ultrasound parameter is

$$20 * \log(\Delta a) = L\alpha \qquad\qquad Eq. \; Z.1$$

wherein $\alpha$ represents the attenuation image of the tissue, i.e. attenuation values assigned to the discrete cells the tissue is virtualized in,

wherein $\Delta a$ represents the difference in amplitudes as per Fig. 8b),

wherein L again is a matrix including weighted path lengths geometric information that depends on the transmitter-receiver setup of the transducer and the size and shape of the cells, in particular their granularity / resolution.

**Claims**

1. Medical ultrasound system, comprising

   - an ultrasound transducer (1) for emitting and receiving ultrasound, and
   - a processor (51),
   wherein the ultrasound transducer (1) is electrically connected to the processor (51), and
   wherein the processor (51) is configured to determine an ultrasound based tomographic image by means of speed of sound and acoustic attenuation reconstruction subject to ultrasound waves (usr) received by the ultrasound transducer (1) in response to ultrasound waves emitted by the ultrasound transducer (1) and scattered and / or reflected by tissue to be investigated.

2. Medical ultrasound system according to claim 1,
   absent a man-made reflector for reflecting the emitted ultrasound waves.

3. Medical ultrasound system according to claim 1 or claim 2,
   wherein the ultrasound transducer (1) is a handheld ultrasound apparatus (10).

4. Medical ultrasound system according to any of the preceding claims,

   wherein the ultrasound transducer (1) comprises a set of emitter elements (Tx) and a set of receiver elements (Rx),
   wherein the processor (51) is configured to, for a set of emitter element/s (Tx) - receiver element/s (Rx) combinations, trigger the respective emitter element/s (Tx) to emit an ultrasound wave (usr),
   wherein the processor (51) is configured to, for each of the emitter element/s (Tx) - receiver element/s (Rx) combinations of the set, determine a time of flight value ($t_p$) or an amplitude or an amplitude measurement (ap) for the ultrasound wave (usr) travelling from the emitter element/s (Tx) to the receiver element/s (Rx),

wherein the processor (51) is configured to determine ultrasound parameter values ($\sigma,\alpha$) of the ultrasound wave (usr) for cells ($c_{i,j}$) in a plane (x,y) defined by the emitted ultrasound wave dependent on the time of flight values ($t_p$) or the amplitudes or the amplitude measurements (ap), and

wherein the processor (51) is configured to convert the ultrasound parameter values ($\sigma,\alpha$) into the image.

5. Medical ultrasound system according to claim 4,

wherein the processor (51) is configured to determine the ultrasound parameter values ($\sigma,\alpha$) dependent on a difference of the time of flight values ($t_p$) for different ultrasound wave propagation paths (p) defined by different emitter element/s (Tx) - receiver element/s (Rx) combinations of the set, and

in particular wherein the ultrasound parameter ($\sigma,\alpha$) is speed of sound ($\sigma$).

6. Medical ultrasound system according to claim 4 or claim 5,

wherein the processor (51) is configured to solve a system of equations which relates a discrete set of time measurements to the speed of sound ($\alpha$) values in a discrete number of the cells ($c_{i,j}$),

in particular wherein a time measurement of the set represents a difference of the time of flight values ($t_p$) for different ultrasound wave propagation paths (p),

in particular wherein the processor (51) is configured to determine the speed of sound values ($\sigma$) for the cells ($c_{i,j}$) by solving the system of equations.

7. Medical ultrasound system according to claim 5 or claim 6,

wherein the processor (51) is configured to calculate speed of sound variations in each of the cells ($c_{i,j}$) of the set from discretized relations between speed of sound increments in individual cells ($c_{i,j}$) of the set and the cumulative time measurements recorded by the transducer (1), given defined propagation paths (p).

8. Medical ultrasound system according to claim 6 or claim 7,

wherein the time measurements are expressed by a linear combination of the speed of sound values in the discrete number of cells.

9. Medical ultrasound system according to any of the preceding claims 5 to 8,

wherein the ultrasound wave propagation paths (p) of the set all convert to or traverse a common point or region in the insonified space, or

wherein the ultrasound wave has a diverging wavefront, or

wherein the ultrasound wave has a circular wavefront.

10. Medical ultrasound system according to any one of the preceding claims 4 to 9,

wherein the processor (51) is configured to determine the ultrasound parameter values ($\sigma,\alpha$) out of a set of ultrasound parameter values dependent on gradients of ultrasound parameter values ($\sigma,\alpha$) of neighboring cells ($c_{i,j}$).

11. Medical ultrasound system according to any one of the preceding claims 4 to 10,

wherein the processor (51) is configured to determine the ultrasound parameter values ($\sigma,\alpha$) out of a set of ultrasound parameter values dependent on gradients of ultrasound parameter values of neighboring cells ($c_{i,j}$) in at least two directions in the plane (x,y).

12. Medical ultrasound system according to claim 11,

wherein the processor (51) is configured to determine the ultrasound parameter values ($\sigma,\alpha$) out of the set of ultrasound parameter values dependent on gradients of ultrasound parameter values in a first direction in the plane (x,y), and dependent on a gradient of ultrasound parameter values in a second direction in the plane (x,y) different to the first direction.

13. Medical ultrasound system according to claim 12,

wherein the first direction is a direction (y) orthogonal to a longitudinal extension of the set of emitter elements (Tx) and the set of receiver elements (Rx), and wherein the second direction (x) is orthogonal to the first direction (y),

preferably wherein the emitter elements of the set and the receiver elements of the set are arranged in a straight row.

**14.** Medical ultrasound system according to claim 12,

wherein each transducer element including one emitter and one receiver element has a principal direction referred to as radiation axis along which the transducer element emits ultrasound,
wherein the first direction represents (y) an average of the radiation axes of the set of emitter elements (Tx) and the set of receiver elements (Rx),
wherein the second direction (x) is a direction (y) orthogonal to a longitudinal extension of the set of emitter elements (Tx) and the set of receiver elements (Rx), and wherein the second direction (x) is orthogonal to the first direction (y),
preferably wherein the emitter elements of the set and the receiver elements of the set are arranged in a curved line, and preferably in a convex line.

**15.** Medical ultrasound system according to any one of the preceding claims 12 or claim 14,
wherein the processor (51) is configured to determine the ultrasound parameter values $(\sigma,\alpha)$ out of the set of ultrasound parameter values in addition dependent on gradients of ultrasound parameter values in a third direction in the plane (x,y), preferably different to the first and second direction.

**16.** Medical ultrasound system according to claim 15,

wherein the second direction is defined by a maximum angle $(\phi_{max})$ with respect to the first direction, whichis defined by a maximum inclination of the ultrasound beam or non-focused ultrasound wavefront transmitted by the set of emitter elements (Tx) and received by the set of receiver elements (Rx),
wherein the third direction is defined by the negative maximum angle $(\phi_{max})$.

**17.** Medical ultrasound according to any one of the preceding claims 3 to 16,
wherein the processor (51) is configured to determine the ultrasound parameter values $(\sigma,\alpha)$ out of a set of ultrasound parameter values dependent on weighted gradients of ultrasound parameter values of neighboring cells $(c_{i,j})$ in at least two directions in the plane (x,y).

**18.** Medical ultrasound system according to claim 17,
wherein the processor (51) is configured to apply the same weight (k) to all gradients of the same direction, and different weights (k, 1-k) per direction.

**19.** Medical ultrasound system according to any one of the preceding claims 12 to 18,
wherein the processor (51) is configured to apply a first weight (k) to all gradients of the first direction, and a second weight (1-k) to all gradients of the second direction, wherein the first weight (k) exceeds the second weight (1-k).

**20.** Medical ultrasound system according to any one of the preceding claims 12 to 19,
wherein the processor (51) is configured to apply a first weight to all gradients of the first direction, a second weight to all gradients of the second direction, and a third weight to all gradients of the third direction.

**21.** Medical ultrasound system according to any one of the preceding claims,
wherein the processor (51) is configured to determine the speed of sound value $(\sigma)$ for each cell $(c_{i,j})$ either as a single value, or in function of the frequency, or in function of any perturbation applied to the tissue.

**22.** Medical ultrasound system according to any of the preceding claims,

wherein the ultrasound parameter $(\sigma,\alpha)$ is acoustic attenuation $(\alpha)$,
wherein the processor (51) is configured to determine an acoustic attenuation value $(\alpha)$ for each cell $(c_{i,j})$,
preferably either as a single value, or in function of the frequency, or in function of any perturbation applied to the tissue.

**23.** Medical ultrasound system according to any of the preceding claims 4 to 22,
wherein the ultrasound parameter values $(\sigma,\alpha)$ are identified at several emitted ultrasound frequencies allowing to reconstruct frequency-dependence of such parameter.

**24.** Medical ultrasound system according to any of the preceding claims,
wherein the processor (51) is configured to determine a distance between the transducer (1) and a structure (m) in the tissue dependent on a time of flight value ($t_p$) in response to triggering an ultrasound wave at the transducer (1).

**25.** Medical ultrasound system according to any of the preceding claims,

wherein the processing unit is configured to apply total-variation regularization in the calculation of tomographic ultrasound images, and
in particular wherein in the total variation regularization the equation to be solved follows the form argmin_o {$\|\Delta t - L\sigma\|$_p + $\lambda\|D$ $\sigma\|$_q} or any combination of such forms, where $\Delta t$ is a vector of measured quantities, $\sigma$ the unknown vector to be reconstructed, L a matrix geometrically calculated under consideration of the setup geometry, D a gradient matrix and $\lambda$ a constant, p and q are orders of the respective norms, and
in particular wherein the equation is solved with convex optimization,
in particular wherein p = 1 and q = 1.

**26.** Medical ultrasound system according to any of the preceding claims 4 to 25,

comprising operating the ultrasound transducer by emitting an ultrasound wave by a single emitter element and the receiver elements receiving the emitted and scattered and / or reflected ultrasound wave,
and preferably operating the ultrasound transducer such that subsequently each emitter element emits an ultrasound wave and the receiver elements receive the emitted and scattered and / or reflected ultrasound wave.

**27.** Method for determining an ultrasound based tomographic image, comprising

emitting ultrasound by an ultrasound transducer (1) towards tissue to be investigated, and receiving ultrasound scattered and / or reflected by tissue to be investigated by the ultrasound transducer (1), and
determining the ultrasound based tomographic image by means of speed of sound and acoustic attenuation reconstruction subject to the ultrasound waves (usr) received by the ultrasound transducer (1) by a processor (51).

**28.** Method for determining an ultrasound based tomographic image according to claim 27, comprising

collecting a plurality of ultrasound signals to allow access to a set of points in an imaged domain from paths passing through different domain regions,
determining local mis-registrations between these plurality of ultrasound signals, and
employing a spatial-domain reconstruction based on the computed mis-registrations in a discretized problem space, preferably in the discretized tissue to be investigated, and
preferably determining the ultrasound based tomographic image from the spatial-domain reconstruction for the discretized problem space.

**Patentansprüche**

**1.** Medizinisches Ultraschallsystem, umfassend

- einen Ultraschallwandler (1) zum Senden und Empfangen von Ultraschall, und
- einen Prozessor (51),
wobei der Ultraschallwandler (1) elektrisch mit dem Prozessor (51) verbunden ist, und
wobei der Prozessor (51) so konfiguriert ist, dass er ein auf Ultraschall basierendes tomographisches Bild mittels Schallgeschwindigkeit und akustischer Dämpfungsrekonstruktion in Abhängigkeit von Ultraschallwellen (usr) bestimmt, die vom Ultraschallwandler (1) als Reaktion auf Ultraschallwellen, die vom Ultraschallwandler (1) gesendet und von dem zu untersuchenden Gewebe gestreut und/oder reflektiert werden, empfangen werden.

**2.** Medizinisches Ultraschallsystem nach Anspruch 1 ohne einen künstlichen Reflektor zum Reflektieren der gesendeten Ultraschallwellen.

**3.** Medizinisches Ultraschallsystem nach Anspruch 1 oder Anspruch 2,
wobei der Ultraschallwandler (1) ein handgehaltenes Ultraschallgerät (10) ist.

4. Medizinisches Ultraschallsystem nach einem der vorangehenden Ansprüche,

wobei der Ultraschallwandler (1) einen Satz von Sendeelementen (Tx) und einen Satz von Empfangselementen (Rx) umfasst,
wobei der Prozessor (51) so konfiguriert ist, dass er für einen Satz von Sendeelementen (Tx) - Empfangselement(en) (Rx) Kombinationen das/die jeweilige(n) Sendeelement(e) (Tx) zum Aussenden einer Ultraschallwelle (usr) ansteuert,
wobei der Prozessor (51) so konfiguriert ist, dass er für jede der Kombinationen aus Sendeelement/en (Tx) und Empfangselement/en (Rx) des Satzes einen Laufzeitwert (tp) oder eine Amplitude oder ein Amplitudenmass (ap) für die Ultraschallwelle (usr) bestimmt, die sich von dem/den Sendeelement/en (Tx) zu dem/den Empfangselement/en (Rx) ausbreitet,
wobei der Prozessor (51) so konfiguriert ist, dass er Ultraschallparameterwerte ($\sigma$, $\alpha$) der Ultraschallwelle (usr) für Zellen ($c_{i,j}$) in einer durch die ausgesendete Ultraschallwelle definierten Ebene (x, y) in Abhängigkeit von den Laufzeitwerten (tp) oder den Amplituden oder den Amplitudenmessungen (ap) bestimmt, und
wobei der Prozessor (51) so konfiguriert ist, dass er die Ultraschallparameterwerte ($\sigma$,$\alpha$) in das Bild umwandelt.

5. Medizinisches Ultraschallsystem nach Anspruch 4,

wobei der Prozessor (51) so konfiguriert ist, dass er die Ultraschallparameterwerte ($\sigma$,$\alpha$) in Abhängigkeit von einer Differenz der Laufzeitwerte (tp) für verschiedene Ultraschallwellenausbreitungswege (p) bestimmt, die durch verschiedene Sendeelement/e (Tx) - Empfangselement/e (Rx) Kombinationen des Satzes definiert sind, und
insbesondere wobei der Ultraschallparameter ($\sigma$, $\alpha$) die Schallgeschwindigkeit ($\sigma$) ist.

6. Medizinisches Ultraschallsystem nach Anspruch 4 oder Anspruch 5,

wobei der Prozessor (51) so konfiguriert ist, dass er ein Gleichungssystem löst, das einen diskreten Satz von Zeitmessungen mit den Werten der Schallgeschwindigkeit ($\sigma$) in einer diskreten Anzahl der Zellen ($c_{i,j}$) in Beziehung setzt,
wobei insbesondere eine Zeitmessung des Satzes eine Differenz der Laufzeitwerte (tp) für verschiedene Ultraschallwellenausbreitungswege (p) darstellt,
insbesondere wobei der Prozessor (51) so konfiguriert ist, dass er die Werte der Schallgeschwindigkeit ($\sigma$) für die Zellen ($c_{i,j}$) durch Lösen des Gleichungssystems bestimmt.

7. Medizinisches Ultraschallsystem nach Anspruch 5 oder Anspruch 6,
wobei der Prozessor (51) so konfiguriert ist, dass er aus diskretisierten Beziehungen zwischen Schallgeschwindigkeitsinkrementen in einzelnen Zellen ($c_{i,j}$) des Satzes und den vom Ultraschallwandler (1) aufgenommenen kumulativen Zeitmessungen bei definierten Ausbreitungswegen (p) Schallgeschwindigkeitsänderungen in jeder der Zellen ($c_{i,j}$) des Satzes zu berechnen.

8. Medizinisches Ultraschallsystem nach Anspruch 6 oder Anspruch 7,
wobei die Zeitmessungen durch eine Linearkombination der Werte der Schallgeschwindigkeit in der diskreten Anzahl von Zellen ausgedrückt sind.

9. Medizinisches Ultraschallsystem nach einem der vorangehenden Ansprüche 5 bis 8,

wobei die Ultraschallwellenausbreitungswege (p) des Satzes alle in einen gemeinsamen Punkt oder Bereich im beschallten Raum übergehen oder diesen durchqueren, oder
wobei die Ultraschallwelle eine divergierende Wellenfront aufweist, oder
wobei die Ultraschallwelle eine kreisförmige Wellenfront aufweist.

10. Medizinisches Ultraschallsystem nach einem der vorangehenden Ansprüche 4 bis 9,
wobei der Prozessor (51) so konfiguriert ist, dass er die Ultraschallparameterwerte ($\sigma$, $\alpha$) aus einem Satz von Ultraschallparameterwerten in Abhängigkeit von Gradienten von Ultraschallparameterwerten ($\sigma$, $\alpha$) benachbarter Zellen ($c_{i,j}$) bestimmt.

11. Medizinisches Ultraschallsystem nach einem der vorangehenden Ansprüche 4 bis 10,
wobei der Prozessor (51) so konfiguriert ist, dass er die Ultraschallparameterwerte ($\sigma$, $\alpha$) aus einem Satz von Ultraschallparameterwerten in Abhängigkeit von Gradienten von Ultraschallparameterwerten benachbarter Zellen ($c_{i,j}$) in mindestens zwei Richtungen in der Ebene (x,y) bestimmt.

12. Medizinisches Ultraschallsystem nach Anspruch 11,
wobei der Prozessor (51) so konfiguriert ist, dass er die Ultraschallparameterwerte ($\sigma, \alpha$) aus dem Satz von Ultraschallparameterwerten in Abhängigkeit von Gradienten von Ultraschallparameterwerten in einer ersten Richtung in der Ebene (x, y) und in Abhängigkeit von einem Gradienten von Ultraschallparameterwerten in einer zweiten Richtung in der Ebene (x, y), die sich von der ersten Richtung unterscheidet, bestimmt.

13. Medizinisches Ultraschallsystem nach Anspruch 12,

wobei die erste Richtung eine Richtung (y) orthogonal zu einer Längserstreckung des Satzes von Sendeelementen (Tx) und des Satzes von Empfangselementen (Rx) ist, und wobei die zweite Richtung (x) orthogonal zur ersten Richtung (y) ist,
wobei vorzugsweise die Sendeelemente des Satzes und die Empfangselemente des Satzes in einer geraden Reihe angeordnet sind.

14. Medizinisches Ultraschallsystem nach Anspruch 12,

wobei jedes Wandlerelement mit einem Sende- und einem Empfangselement eine als Strahlungsachse bezeichnete Hauptrichtung aufweist, entlang der das Wandlerelement Ultraschall sendet,
wobei die erste Richtung (y) einen Durchschnitt der Strahlungsachsen des Satzes von Sendeelementen (Tx) und des Satzes von Empfangselementen (Rx) darstellt,
wobei die zweite Richtung (x) eine Richtung (y) orthogonal zu einer Längserstreckung des Satzes von Sendeelementen (Tx) und des Satzes von Empfangselementen (Rx) ist, und wobei die zweite Richtung (x) orthogonal zur ersten Richtung (y) ist,
wobei die Senderelemente des Satzes und die Empfangselemente des Satzes vorzugsweise in einer gekrümmten Linie und vorzugsweise in einer konvexen Linie angeordnet sind.

15. Medizinisches Ultraschallsystem nach einem der vorangehenden Ansprüche 12 oder 14,
wobei der Prozessor (51) so konfiguriert ist, dass er die Ultraschallparameterwerte ($\sigma, \alpha$) aus dem Satz von Ultraschallparameterwerten zusätzlich in Abhängigkeit von Gradienten von Ultraschallparameterwerten in einer dritten, vorzugsweise von der ersten und zweiten Richtung verschiedenen Richtung in der Ebene (x, y) zu bestimmen.

16. Medizinisches Ultraschallsystem nach Anspruch 15,

wobei die zweite Richtung durch einen maximalen Winkel ($\phi_{max}$) in Bezug auf die erste Richtung definiert ist, der durch eine maximale Neigung des Ultraschallstrahls oder der nicht fokussierten Ultraschallwellenfront definiert ist, die vom Satz von Sendeelementen (Tx) gesendet und vom Satz von Empfangselementen (Rx) empfangen wird,
wobei die dritte Richtung durch den negativen Maximalwinkel ($\phi_{max}$) definiert ist.

17. Medizinisches Ultraschallsystem nach einem der vorangehenden Ansprüche 3 bis 16,
wobei der Prozessor (51) so konfiguriert ist, dass er die Ultraschallparameterwerte ($\sigma, \alpha$) aus einem Satz von Ultraschallparameterwerten in Abhängigkeit von gewichteten Gradienten von Ultraschallparameterwerten benachbarter Zellen ($c_{i,j}$) in mindestens zwei Richtungen in der Ebene (x, y) bestimmt.

18. Medizinisches Ultraschallsystem nach Anspruch 17,
wobei der Prozessor (51) so konfiguriert ist, dass er auf alle Gradienten der gleichen Richtung die gleiche Gewichtung (k) und auf jede Richtung unterschiedliche Gewichtungen (k, 1-k) anwendet.

19. Medizinisches Ultraschallsystem nach einem der vorangehenden Ansprüche 12 bis 18,
wobei der Prozessor (51) so konfiguriert ist, dass er eine erste Gewichtung (k) auf alle Gradienten der ersten Richtung und eine zweite Gewichtung (1-k) auf alle Gradienten der zweiten Richtung anwendet, wobei die erste Gewichtung (k) die zweite Gewichtung (1-k) übersteigt.

20. Medizinisches Ultraschallsystem nach einem der vorangehenden Ansprüche 12 bis 19,
wobei der Prozessor (51) so konfiguriert ist, dass er eine erste Gewichtung auf alle Gradienten der ersten Richtung, eine zweite Gewichtung auf alle Gradienten der zweiten Richtung und eine dritte Gewichtung auf alle Gradienten der dritten Richtung anwendet.

21. Medizinisches Ultraschallsystem nach einem der vorangehenden Ansprüche,
wobei der Prozessor (51) so konfiguriert ist, dass er den Wert der Schallgeschwindigkeit ($\sigma$) für jede Zelle ($c_{i,j}$) entweder als Einzelwert oder in Abhängigkeit von der Frequenz oder in Abhängigkeit von einer auf das Gewebe ausgeübten Störung bestimmt.

22. Medizinisches Ultraschallsystem nach einem der vorangehenden Ansprüche,

wobei der Ultraschallparameter ($\sigma$, $\alpha$) die akustische Dämpfung ($\alpha$) ist,
wobei der Prozessor (51) so konfiguriert ist, dass er einen akustischen Dämpfungswert ($\alpha$) für jede Zelle ($c_{i,j}$) bestimmt, vorzugsweise entweder als Einzelwert oder in Abhängigkeit von der Frequenz oder in Abhängigkeit von einer auf das Gewebe einwirkenden Störung.

23. Medizinisches Ultraschallsystem nach einem der vorangehenden Ansprüche 4 bis 22,
wobei die Werte der Ultraschallparameter ($\sigma$, $\alpha$) bei mehreren gesendeten Ultraschallfrequenzen identifiziert werden, was es ermöglicht, die Frequenzabhängigkeit eines solchen Parameters zu rekonstruieren.

24. Medizinisches Ultraschallsystem nach einem der vorangehenden Ansprüche,
wobei der Prozessor (51) so konfiguriert ist, dass er als Reaktion auf die Auslösung einer Ultraschallwelle am Wandler (1) einen Abstand zwischen dem Wandler (1) und einer Struktur (m) im Gewebe in Abhängigkeit von einem Laufzeitwert (tp) bestimmt.

25. Medizinisches Ultraschallsystem nach einem der vorangehenden Ansprüche,

wobei die Prozessoreinheit so konfiguriert ist, dass sie bei der Berechnung von tomographischen Ultraschallbildern eine Regularisierung der Gesamtvariation anwendet, und
insbesondere wobei bei der Regularisierung der Gesamtvariation die zu lösende Gleichung der Form argmin_$\sigma$ $\{\|\Delta t - L\sigma\|\_p + \lambda\|D\ \sigma\|\_q\}$ oder einer beliebigen Kombination solcher Formen folgt, wobei $\Delta t$ ein Vektor von Messgrössen ist, $\sigma$ der zu rekonstruierende unbekannte Vektor, L eine geometrisch unter Berücksichtigung der Aufstellungsgeometrie berechnete Matrix, D eine Gradientenmatrix und $\lambda$ eine Konstante sind, p und q Ordnungen der jeweiligen Normen sind, und
insbesondere wobei die Gleichung mit konvexer Optimierung gelöst wird,
insbesondere wobei p = 1 und q = 1 ist.

26. Medizinisches Ultraschallsystem nach einem der vorangehenden Ansprüche 4 bis 25,

wobei der Ultraschallwandler durch Senden einer Ultraschallwelle durch ein einzelnes Sendeelement betrieben wird und die Empfangselemente die gesendete und gestreute und/oder reflektierte Ultraschallwelle empfangen, und vorzugsweise den Ultraschallwandler so zu betreiben, dass anschliessend jedes Sendeelement eine Ultraschallwelle sendet und die Empfangselemente die gesendete und gestreute und/oder reflektierte Ultraschallwelle empfangen.

27. Verfahren zur Bestimmung eines tomographischen Bildes auf Ultraschallbasis, umfassend

Senden von Ultraschall durch einen Ultraschallwandler (1) in Richtung des zu untersuchenden Gewebes und Empfangen des von dem zu untersuchenden Gewebe gestreuten und/oder reflektierten Ultraschalls durch den Ultraschallwandler (1), und
Bestimmen des ultraschallbasierten tomographischen Bildes mittels Schallgeschwindigkeit und akustischer Dämpfungsrekonstruktion in Abhängigkeit von den durch den Ultraschallwandler (1) empfangenen Ultraschallwellen (usr) durch einen Prozessor (51).

28. Verfahren zur Bestimmung eines ultraschallbasierten tomographischen Bildes nach Anspruch 27, umfassend

Sammeln einer Vielzahl von Ultraschallsignalen, um Zugang zu einem Satz von Punkten in einer abgebildeten Domäne von Pfaden zu ermöglichen, die durch verschiedene Domänenbereiche verlaufen,
Bestimmen lokaler Fehlregistrierungen zwischen dieser Vielzahl von Ultraschallsignalen, und

**EP 3 577 491 B1**

Anwenden einer räumlichen Bereichsrekonstruktion auf der Grundlage der berechneten Fehlregistrierungen in einem diskretisierten Problemraum, vorzugsweise in dem zu untersuchenden diskretisierten Gewebe, und vorzugsweise Bestimmen des ultraschallbasierten tomographischen Bildes aus der räumlichen Bereichsrekonstruktion für den diskretisierten Problemraum.

**Revendications**

1. Système médical à ultrasons comprenant

   - un transducteur à ultrasons (1) pour émettre et recevoir des ultrasons, et
   - un processeur (51),
   dans lequel le transducteur à ultrasons (1) est connecté électriquement au processeur (51), et
   dans lequel le processeur (51) est configuré pour déterminer une image tomographique basée sur les ultrasons au moyen de la vitesse du son et de la reconstruction de l'atténuation acoustique sous réserve des ondes ultrasonores (usr) reçues par le transducteur à ultrasons (1) en réponse aux ondes ultrasonores émises par le transducteur à ultrasons (1) et diffusées et/ou réfléchies par le tissu à examiner.

2. Système médical à ultrasons selon la revendication 1,
   sans réflecteur artificiel pour réfléchir les ondes ultrasonores émises.

3. Système médical à ultrasons selon la revendication 1 ou la revendication 2,
   dans lequel le transducteur à ultrasons (1) est un appareil à ultrasons portatif (10).

4. Système médical à ultrasons selon l'une des revendications précédentes,

   dans lequel le transducteur à ultrasons (1) comprend un ensemble d'éléments émetteurs (Tx) et un ensemble d'éléments récepteurs (Rx),
   dans lequel le processeur (51) est configuré, pour un ensemble de combinaisons élément émetteur (Tx) - élément récepteur (Rx), pour déclencher l'élément émetteur respectif (Tx) pour émettre une onde ultrasonore (usr),
   dans lequel le processeur (51) est configuré, pour chacune des combinaisons élément émetteur (Tx) - élément récepteur (Rx) de l'ensemble, pour déterminer une valeur de temps de parcours (tp) ou une amplitude ou une mesure d'amplitude (ap) pour l'onde ultrasonore (usr) se propageant de l'élément émetteur (Tx) à l'élément récepteur (Rx),
   dans lequel le processeur (51) est configuré pour déterminer des valeurs des paramètres ultrasonores $(\sigma, \alpha)$ de l'onde ultrasonore (usr) pour les cellules $(c_{i,j})$ dans un plan (x, y) défini par l'onde ultrasonore émise en fonction des valeurs de temps de parcours (tp) ou des amplitudes ou des mesures d'amplitude (ap), et
   dans lequel le processeur (51) est configuré pour convertir les valeurs des paramètres ultrasonores $(\sigma, \alpha)$ en l'image.

5. Système médical à ultrasons selon la revendication 4,

   dans lequel le processeur (51) est configuré pour déterminer les valeurs des paramètres ultrasonores $(\sigma, \alpha)$ en fonction d'une différence des valeurs de temps de parcours (tp) pour différents trajets de propagation des ondes ultrasonores (p) définis par différentes combinaisons élément émetteur/s (Tx) - élément récepteur/s (Rx) de l'ensemble, et
   en particulier, dans lequel le paramètre ultrasonore $(\sigma, \alpha)$ est la vitesse du son $(\sigma)$.

6. Système médical à ultrasons selon la revendication 4 ou la revendication 5,

   dans lequel le processeur (51) est configuré pour résoudre un système d'équations qui relie un ensemble discret de mesures temporelles aux valeurs de la vitesse du son $(\sigma)$ dans un nombre discret de cellules $(c_{i,j})$,
   en particulier, dans lequel une mesure temporelle de l'ensemble représente une différence des valeurs de temps de parcours (tp) pour différents chemins de propagation d'ondes ultrasonores (p),
   en particulier, le processeur (51) étant configuré pour déterminer les valeurs de vitesse du son $(\sigma)$ pour les cellules $(c_{i,j})$ en résolvant le système d'équations.

**7.** Système médical à ultrasons selon la revendication 5 ou la revendication 6,
dans lequel le processeur (51) est configuré pour calculer les variations de la vitesse du son dans chacune des cellules ($c_{i,j}$) de l'ensemble à partir de relations discrétisées entre les incréments de vitesse du son dans les cellules individuelles ($c_{i,j}$) de l'ensemble et les mesures de temps cumulées enregistrées par le transducteur (1), étant donné des trajets de propagation définis (p).

**8.** Système médical à ultrasons selon la revendication 6 ou la revendication 7,
dans lequel les mesures de temps sont exprimées par une combinaison linéaire des valeurs de vitesse du son dans le nombre discret de cellules.

**9.** Système médical à ultrasons selon l'une des revendications précédentes 5 à 8,

dans lequel les trajets de propagation de l'onde ultrasonore (p) de l'ensemble convergent tous vers un point ou une région commune de l'espace insonifié ou le traversent, ou
dans lequel l'onde ultrasonore a un front d'onde divergent, ou
dans lequel l'onde ultrasonore a un front d'onde circulaire.

**10.** Système médical à ultrasons selon l'une des revendications précédentes 4 à 9,
dans lequel le processeur (51) est configuré pour déterminer les valeurs des paramètres ultrasonores ($\sigma, \alpha$) à partir d'un ensemble de valeurs de paramètres ultrasonores dépendant des gradients des valeurs des paramètres ultrasonores ($\sigma, \alpha$) des cellules voisines ($c_{i,j}$).

**11.** Système médical à ultrasons selon l'une des revendications précédentes 4 à 10,
dans lequel le processeur (51) est configuré pour déterminer les valeurs des paramètres ultrasonores ($\sigma, \alpha$) parmi un ensemble de valeurs de paramètres ultrasonores dépendant des gradients des valeurs des paramètres ultrasonores des cellules voisines ($c_{i,j}$) dans au moins deux directions dans le plan (x, y).

**12.** Système médical à ultrasons selon la revendication 11,
dans lequel le processeur (51) est configuré pour déterminer les valeurs des paramètres ultrasonores ($\sigma, \alpha$) parmi l'ensemble des valeurs des paramètres ultrasonores dépendant des gradients des valeurs des paramètres ultrasonores dans une première direction dans le plan (x, y), et dépendant d'un gradient des valeurs des paramètres ultrasonores dans une deuxième direction dans le plan (x, y) différente de la première direction.

**13.** Système médical à ultrasons selon la revendication 12,

dans lequel la première direction est une direction (y) orthogonale à une extension longitudinale de l'ensemble d'éléments émetteurs (Tx) et de l'ensemble d'éléments récepteurs (Rx), et dans lequel la deuxième direction (x) est orthogonale à la première direction (y),
de préférence, dans lequel les éléments émetteurs de l'ensemble et les éléments récepteurs de l'ensemble sont disposés en ligne droite.

**14.** Système médical à ultrasons selon la revendication 12,

dans lequel chaque élément transducteur comprenant un élément émetteur et un élément récepteur a une direction principale appelée axe de rayonnement le long duquel l'élément transducteur émet des ultrasons,
dans lequel la première direction représente (y) une moyenne des axes de rayonnement de l'ensemble des éléments émetteurs (Tx) et de l'ensemble des éléments récepteurs (Rx),
dans lequel la deuxième direction (x) est une direction (y) orthogonale à une extension longitudinale de l'ensemble d'éléments émetteurs (Tx) et de l'ensemble d'éléments récepteurs (Rx), et dans lequel la deuxième direction (x) est orthogonale à la première direction (y),
de préférence, dans lequel les éléments émetteurs de l'ensemble et les éléments récepteurs de l'ensemble sont disposés selon une ligne courbe, et de préférence selon une ligne convexe.

**15.** Système médical à ultrasons selon l'une des revendications précédentes 12 ou 14,
dans lequel le processeur (51) est configuré pour déterminer les valeurs des paramètres ultrasonores ($\sigma, \alpha$) à partir de l'ensemble des valeurs des paramètres ultrasonores en fonction en outre des gradients des valeurs des paramètres ultrasonores dans une troisième direction dans le plan (x, y), de préférence différente de la première et de la deuxième direction.

**16.** Système médical à ultrasons selon la revendication 15,

dans laquelle la deuxième direction est définie par un angle maximal (ϕmax) par rapport à la première direction, qui est défini par une inclinaison maximale du faisceau ultrasonore ou du front d'onde ultrasonore non focalisé transmis par l'ensemble d'éléments émetteurs (Tx) et reçu par l'ensemble d'éléments récepteurs (Rx), dans laquelle la troisième direction est définie par l'angle maximal négatif (ϕmax).

**17.** Système médical à ultrasons selon l'une des revendications précédentes 3 à 16, dans lequel le processeur (51) est configuré pour déterminer les valeurs de paramètres ultrasonores ($\sigma$, $\alpha$) parmi un ensemble de valeurs de paramètres ultrasonores dépendant des gradients pondérés des valeurs de paramètres ultrasonores des cellules voisines ($c_{i,j}$) dans au moins deux directions dans le plan (x, y).

**18.** Système médical à ultrasons selon la revendication 17, dans lequel le processeur (51) est configuré pour appliquer le même poids (k) à tous les gradients de la même direction, et différents poids (k, 1-k) par direction.

**19.** Système médical à ultrasons selon l'une des revendications précédentes 12 à 18, dans lequel le processeur (51) est configuré pour appliquer un premier poids (k) à tous les gradients de la première direction, et un second poids (1-k) à tous les gradients de la seconde direction, le premier poids (k) étant supérieur au second poids (1-k).

**20.** Système médical à ultrasons selon l'une des revendications précédentes 12 à 19, dans lequel le processeur (51) est configuré pour appliquer un premier poids à tous les gradients de la première direction, un deuxième poids à tous les gradients de la deuxième direction et un troisième poids à tous les gradients de la troisième direction.

**21.** Système médical à ultrasons selon l'une des revendications précédentes, dans lequel le processeur (51) est configuré pour déterminer la valeur de la vitesse du son ($\sigma$) pour chaque cellule ($c_{i,j}$) soit en tant que valeur unique, soit en fonction de la fréquence, soit en fonction de toute perturbation appliquée au tissu.

**22.** Système médical à ultrasons selon l'une des revendications précédentes,

dans lequel le paramètre ultrasonore ($\sigma$, $\alpha$) est l'atténuation acoustique ($\alpha$), dans lequel le processeur (51) est configuré pour déterminer une valeur d'atténuation acoustique ($\alpha$) pour chaque cellule ($c_{i,j}$), de préférence sous forme de valeur unique, ou en fonction de la fréquence, ou en fonction de toute perturbation appliquée au tissu.

**23.** Système médical à ultrasons selon l'une des revendications précédentes 4 à 22, dans lequel les valeurs des paramètres ultrasonores ($\sigma$, $\alpha$) sont identifiées à plusieurs fréquences ultrasonores émises, ce qui permet de reconstruire la dépendance en fréquence de ces paramètres.

**24.** Système médical à ultrasons selon l'une des revendications précédentes, dans lequel le processeur (51) est configuré pour déterminer une distance entre le transducteur (1) et une structure (m) dans le tissu en fonction d'une valeur de temps de parcours (tp) en réponse au déclenchement d'une onde ultrasonore au niveau du transducteur (1).

**25.** Système médical à ultrasons selon l'une des revendications précédentes,

dans lequel l'unité de traitement est configurée pour appliquer une régularisation par variation totale dans le calcul des images ultrasonores tomographiques, et en particulier dans lequel, dans la régularisation par variation totale, l'équation à résoudre suit la forme $\text{argmin}\_\sigma$ $\{\|\Delta t - L\sigma\|\_p + \lambda\|D\sigma\|\_q\}$ ou toute combinaison de telles formes, où $\Delta t$ est un vecteur de quantités mesurées, $\sigma$ le vecteur inconnu à reconstruire, L une matrice calculée géométriquement en tenant compte de la géométrie du montage, D une matrice de gradient et $\lambda$ une constante, p et q étant les ordres des normes respectives, et en particulier dans lequel l'équation est résolue par optimisation convexe, en particulier dans laquelle p = 1 et q = 1.

**26.** Système médical à ultrasons selon l'une des revendications précédentes 4 à 25,

comprenant le fonctionnement du transducteur à ultrasons par l'émission d'une onde ultrasonore par un seul élément émetteur et les éléments récepteurs recevant l'onde ultrasonore émise et diffusée et/ou réfléchie, et de préférence en faisant fonctionner le transducteur à ultrasons de telle sorte que chaque élément émetteur émette ensuite une onde ultrasonore et que les éléments récepteurs reçoivent l'onde ultrasonore émise et diffusée et/ou réfléchie.

**27.** Procédé de détermination d'une image tomographique basée sur les ultrasons, comprenant

emmètre d'ultrasons par un transducteur à ultrasons (1) en direction du tissu à examiner, et la réception d'ultrasons diffusés et/ou réfléchis par le tissu à examiner par le transducteur à ultrasons (1), et déterminer l'image tomographique basée sur les ultrasons en fonction des ondes ultrasonores (usr) reçues par le transducteur à ultrasons (1) par un processeur (51).

**28.** Procédé de détermination d'une image tomographique à base d'ultrasons selon la revendication 27, comprenant

collectionner une pluralité de signaux ultrasonores pour permettre l'accès à un ensemble de points dans un domaine imagé à partir de trajectoires passant par différentes régions du domaine, déterminer les erreurs d'enregistrement locales entre cette pluralité de signaux ultrasonores, et utiliser une reconstruction dans le domaine spatial basée sur les erreurs d'enregistrement calculées dans un espace de problème discrétisé, de préférence dans le tissu discrétisé à étudier, et de préférence, déterminer l'image tomographique basée sur les ultrasons à partir de la reconstruction du domaine spatial pour l'espace de problème discrétisé.

FIG. 1

FIG. 2

c)

d)

FIG. 2 cont.

e)

FIG. 2 cont.

Prior information

Reconstruction setup

θ1  θ2  θ3  θ4

p2  p3

p1  p4

m2

m1

Convergence (prior,recon)?    NO

θ1  θ2  θ3  θ4

p2  p3

p1  p4

m2

m1

Convergence (prior,recon)?    NO

YES

STOP

FIG. 3

EP 3 577 491 B1

6/10

FIG. 5

FIG. 6

FIG. 7

FIG. 4

a)

b)

Fig. 8

## REFERENCES CITED IN THE DESCRIPTION

### Patent documents cited in the description

- WO 2015091519 A1
- WO 2015091519 A

- US 20020099290 A1

### Non-patent literature cited in the description

- **DURIC et al.** Breast density measurements with ultrasound tomography: A comparison with film and digital mammography. *Med. Phys.,* 2013, vol. 40 (1
- **NEBEKER et al.** Imaging of Sound Speed Using Reflection Ultrasound Tomography. *Ultrasound Med,* 2012, vol. 31, 1389-1404
- **JAEGER et al.** Computer ultrasound tomography in echo mode for imaging speed of sound using pulse-echo sonography: proof of principle. *Ultrasound in Med. & Biol.,* 2015, vol. 41 (1), 235-250

- **F. VIOLA ; W. WALKER.** A comparison of the performance of timedelay estimators in medical ultrasound. *IEEE Trans. Ultrason. Ferroelectr. Freq. Control,* April 2003, vol. 50, 392-401
- **REZA ZAHIRI-AZAR ; ORCUN GOKSEL ; SEPTIMIU E. SALCUDEAN.** Sub-sample Displacement Estimation from Digitized Ultrasound RF Signals Using Multi-Dimensional Polynomial Fitting of the Cross-correlation Function. *IEEE Trans Ultrasonics, Ferroelectrics, and Frequency Control,* November 2010, vol. 57 (11), 2403-20
- **TOBIAS GASS ; GABOR SZEKELY ; ORCUN GOKSEL.** Consistency-Based Rectification of Non-Rigid Registrations. *SPIE J Medical Imaging,* May 2015, vol. 2 (1), 014005